# EUROPEAN PATENT SPECIFICATION

(11) **EP 1 490 493 B1**
(45) Date of publication and mention of the grant of the patent: **06.12.2006**
(21) Application number: 03715173.5
(22) Date of filing: 04.04.2003
(51) Int. Cl.: C12N 15/54, C12Q 1/68, G01N 33/50

(54) **ENHANCER SEQUENCE OF THE 5-AMINOLEVULINIC ACID SYNTHASE GENE**
ENHANCER-SEQUENZ DES 5-AMINOLÄVULINSÄURE-SYNTHASE GENS
SEQUENCE ACTIVATRICE DU GENE D'ACIDE 5-AMINOLEVULINIQUE SYNTHASE

(30) Priority: 09.04.2002 WO PCT/IB02/01258
(43) Date of publication of application: 29.12.2004
(73) Proprietor: Universität Basel WTT-Stelle, 4003 Basel (CH)
(72) Inventor: MEYER, Urs, Albert, CH-4104 Oberwil (CH); FRASER, David, John, CH-4102 Biningen (CH); KAUFMANN, Michel, R., CH-4055 Basel (CH); PODVINEC, Michael, CH-4055 Basel (CH); ZUMSTEG, Adrian, CH-4056 Basel (CH)
(86) International application number: PCT/IB2003/001414
(87) International publication number: WO 2003/085113

(56) References cited:
- WO-A-99/61622
- DATABASE EMBL14 April 2000 (2000-04-14) "Homo sapiens chromosome 3 clone RP11-758M15 map 3, WORKING DRAFT SEQUENCE, 64 unordered pieces" Database accession no. AC044892 XP002215331
- DATABASE EMBL 29 December 1998 (1998-12-29) "Homo sapiens 3p21.1 contig 9 PAC RPCI5-1157M23 (Roswell Park Cancer Institute Human PAC Library) complete sequence" Database accession no. AC006252 XP002215332
- DATABASE EMBL24 October 2001 (2001-10-24) "Homo sapiens chromosome 3 clone RP11-330H6, complete sequence" Database accession no. AC097637 XP002215333
- BRAIDOTTI GIOVANNA ET AL: "Identification of regulatory sequences in the gene for 5-aminolevulinate synthase from rat." JOURNAL OF BIOLOGICAL CHEMISTRY, vol. 268, no. 2, 1993, pages 1109-1117, XP002215328 ISSN: 0021-9258
- ROBERTS A G ET AL: "Alternative splicing and tissue-specific transcription of human and rodent ubiquitous 5-aminolevulinate synthase (ALAS1) genes" BIOCHIMICA ET BIOPHYSICA ACTA. GENE STRUCTURE AND EXPRESSION, ELSEVIER, AMSTERDAM, NL, vol. 1518, no. 1-2, 19 March 2001 (2001-03-19), pages 95-105, XP004275860 ISSN: 0167-4781

### SEQUENCE LISTING

<110> University Basel
<120> Enhancer sequence of the 5-aminolevulinic acid synthase gene
<130> 06052PC1
<150> PCT/IB02/01258
   <151> 2002-04-09
<160> 39
<170> PatentIn version 3.1
<210> 1
   <211> 167
   <212> DNA
   <213> Gallus gallus
<400> 1
   ctgcctccag tcctgaactt tctctgctgg gatgagcaga gttcacgctc ggctgaactc     60
   gtgactgtgt caacaggggg catgaagatc agcaccaggc aaaggtgagc ggagtgcaca    120
   agaatgaggc agagaccttt gggacaaaga gttccccacc cgtgggg                   167
<210> 2
   <211> 176
   <212> DNA
   <213> Gallus gallus
<400> 2
   gggctccatc ggcctcttca ggttattgct atgttcaact cgtgtgacct tcctccctgt     60
   tttcaaggtt ctgataacaa acttctagga cagcctggtg acctttggct cagcttcctc    120
   tttaaactcc ggtggctttt gcttcattgc ctagttgtta cccagcagta aagatc        176
<210> 3
   <211> 175
   <212> DNA
   <213> Mus musculus
<400> 3
   tgagctcatc aggttcctgg tggagagctg ggtgaaccga gttcgtttgc actgccttgg     60
   cctgtgtgtg gcttcaggaa caggtcatgc tccaggattt aggacacagg ttagctgata    120
   aggcccatgg agccggtgat atgacccgca gggtcactcc ctctgcccaa gtcta         175
<210> 4
   <211> 280
   <212> DNA
   <213> Mus musculus
<400> 4
   agtccagtca gaaccttctg gcttctgcca tgagacagcc tatgagctca tcaggttcct 60
   ggtggagagc tgggtgaacc gagttcgttt gcactgcctt ggcctgtgtg tggcttcagg 120
   aacaggtcat gctccaggat ttaggacaca ggttagctga taaggcccat ggagccggtg 180
   atatgacccg cagggtcact ccctctgccc aagtctagat acttgtttcc ctctttagac 240
   tccagatcaa ggactccaga tctgtcatgg actcagaaaa 280
<210> 5
   <211> 321
   <212> DNA
   <213> Mus musculus
<400> 5
   agtccagtca gaaccttctg gcttctgcca tgagacagcc tatgagctca tcaggttcct 60
   ggtggagagc tgggtgaacc gagttcgttt gcactgcctt ggcctgtgtg tggcttcagg 120
   aacaggtcat gctccaggat ttaggacaca ggttagctga taaggcccat ggagccggtg 180
   atatgacccg cagggtcact ccctctgccc aagtctagat acttgtttcc ctctttagac 240
   tccagatcaa ggactccaga tctgtcatgg actcagaaaa ccccccagta ccaccccacc 300
   cccgctgctc cctcctatga t 321
<210> 6
   <211> 328
   <212> DNA
   <213> Mus musculus
<400> 6
   gtggctgggt tgggatggga tgggaaggct tgtgtctctc tgattcctag tccagtcaga 60
   accttctggc ttctgccatg agacagccta tgagctcatc aggttcctgg tggagagctg 120
   ggtgaaccga gttcgtttgc actgccttgg cctgtgtgtg gcttcaggaa caggtcatgc 180
   tccaggattt aggacacagg ttagctgata aggcccatgg agccggtgat atgacccgca 240
   gggtcactcc ctctgcccaa gtctagatac ttgtttccct ctttagactc cagatcaagg 300
   actccagatc tgtcatggac tcagaaaa 328
<210> 7
   <211> 369
   <212> DNA
   <213> Mus musculus
<400> 7
   gtggctgggt tgggatggga tgggaaggct tgtgtctctc tgattcctag tccagtcaga 60
   accttctggc ttctgccatg agacagccta tgagctcatc aggttcctgg tggagagctg 120
   ggtgaaccga gttcgtttgc actgccttgg cctgtgtgtg gcttcaggaa caggtcatgc 180
   tccaggattt aggacacagg ttagctgata aggcccatgg agccggtgat atgacccgca 240
   gggtcactcc ctctgcccaa gtctagatac ttgtttccct ctttagactc cagatcaagg 300
   actccagatc tgtcatggac tcagaaaacc ccccagtacc accccacccc cgctgctccc 360
   tcctatgat 369
<210> 8
   <211> 174
   <212> DNA
   <213> Homo sapiens
<400> 8
   gcgcaaagtc aacacaagcc tctccaccgt gtgtccatgt ttatgtgtat gcgctgtgcc 60
   ccgtcatgcc acctggacgc agggactcca gtgacctctc cttgcacaag cctctgctgg 120
   tttgggaaag attggcatga catcagccaa gctctggcct tgcctttttt ccct 174
<210> 9
   <211> 800
   <212> DNA
   <213> Homo sapiens
<400> 9
   caggcgcaaa gtcaacacaa gcctctccac cgtgtgtcca tgtttatgtg tatgcgctgt 60
   gccccgtcat gccacctgga cgcagggact ccagtgacct ctccttgcac aagcctctgc 120
   tggtttggga aagattggca tgacatcagc caagctctgg ccttgccttt tttccctccc 180
   ggaacccggc tggctcaaga tctgagccgt ggatctgcac ccactttggg gagttcctgc 240
   ccttgggcta gagtagaggc caagagtcaa agtgtggtgg gggctgaggc agcaggatcg 300
   cttgagccca ggagttcaag gttagagtta tgtttgcatc actgcattcc agcctgggta 360
   agagagcgag accctgcctc aaaaaaaaaa aaaaaaaaaa aaaaagagtg gtgggggtag 420
   ggacagggag atgaggaagg ccctacagtg gagaaagcac caggaccaga acccagccct 480
   cccttgtctg aatcttgctg cccacaggag cctggacagt ggccagggaa ggttcgaatg 540
   ccacacaggt gagcttggcc tctgctctgt aggcagtggg aagtgctggg agttgggcat 600
   tcctgtgagg cgcatagtca acattgtgag tagggctgga tgtcgagctg tgagagggaa 660
   actagaagct ggaacatcta caggaggctt ttaagagaag cagagcggcc aggtgcagtg 720
   gctgacacct gtaatcccag cactttgggt ggccgagggg gtcagaacac ttgaggtcag 780
   gagttcgaga ccagggtgac 800
<210> 10
   <211> 935
   <212> DNA
   <213> Homo sapiens
<400> 10
   cacggtacca caggaggcaa aggaccaccc atggaatcca cccaggaggg ccaggacagg 60
   ggactcaggg gcctcagcct gcactcacct gctcagcaga gctgagctca gggcataacg 120
   tcagcttcct gggcagaaga gctgccaaaa tcaaagcttt gctagtcaga aaattccttg 180
   ggaatgttga gcaaggccac cactgacatc atgtgcaaat tcgcagacag cctctgcacc 240
   taaggctact cagacccaca gccttggctc gatggggtgg cagactctgt atgccaccag 300
   cacacccacc cacagggcag aggggtcagg acatagaatc agacaggccc cagggacccc 360
   agtgaagatt ataggcagcc attccccact caacagagga gaaggtcaga gccaagtctg 420
   acattccccc atcccctctc cataacaccc atgcatctgg cagtcagaca ggcctaagct 480
   aaacctttcc cccccagcta cccaccaggg tcatccccaa gccaggtcag ggccaatgga 540
   ggttggggtg gagaagacag gcttggccct atttcctgcc caactcagaa ccttctggtt 600
   tctgccacag gatgccttgc aagcttatcg gggtcactgt gggcagctgg gtgagctaag 660
   ttcatctgtg ctgccgtgac ctctgtgcag atgcatcaag aacacagagt gctccggggt 720
   taggatgagg gcagcgctga taaggttcat ggaaccagtg acagagcaca cagctgccca 780
   cagagtcact cccctgtgcc ccagcctgga cacctcagct ccctctcaac cccttcccga 840
   ggtgctagat gtatatggga ccagaaagcc ccctctgtgt cctcctgtgt gagagcccag 900
   ctgcttaggt gtttgtgact ctgggcctcg agggg 935
<210> 11
   <211> 25
   <212> DNA
   <213> Artificial sequence
<220>
   <223> Forward PCR primer
<400> 11
   ggggaccagt ccagtcagaa ccttc 25
<210> 12
   <211> 27
   <212> DNA
   <213> Artificial sequence
<220>
   <223> Reverse PCR primer
<400> 12
   ccgctcgagt tttctgagtc catgaca 27
<210> 13
   <211> 26
   <212> DNA
   <213> Artificial sequence
<220>
   <223> Forward PCR primer
<400> 13
   ggggtaccgt ggctgggttg ggatgg 26
<210> 14
   <211> 27
   <212> DNA
   <213> Artificial sequence
<220>
   <223> Reverse PCR primer
<400> 14
   ccgctcgaga tcataggagg gagcagc 27
<210> 15
   <211> 32
   <212> DNA
   <213> artificial sequence
<220>
   <223> forward PCR primer
<400> 15
   ggaggaactc gacacgatac caacatagca at 32
<210> 16
   <211> 30
   <212> DNA
   <213> artificial sequence
<220>
   <223> reverse PCR primer
<400> 16
   ctatgttggt atcgtgtcga gttcctccct 30
<210> 17
   <211> 28
   <212> DNA
   <213> artificial sequence
<220>
   <223> forward PCR primer
<400> 17
   gaattcgcca actgcagcca ggctgtcc 28
<210> 18
   <211> 30
   <212> DNA
   <213> artificial sequence
<220>
   <223> reverse PCR primer
<400> 18
   cagcctggct gcagttggcg aattctcctc 30
<210> 19
   <211> 33
   <212> DNA
   <213> artificial sequence
<220>
   <223> forward PCR primer
<400> 19
   ccccacgcag ccccaccgct cggctgaact cgt 33
<210> 20
   <211> 34
   <212> DNA
   <213> artificial sequence
<220>
   <223> reverse PCR primer
<400> 20
   gtggggctgc gtggggcagc agagaaagtt cagg 34
<210> 21
   <211> 27
   <212> DNA
   <213> artificial sequence
<220>
   <223> forward PCR primer
<400> 21
   gaattcacag ccatggtgaa gatcagc 27
<210> 22
   <211> 25
   <212> DNA
   <213> artificial sequence
<220>
   <223> reverse PCR primer
<400> 22
   ccatggctgt gaattcagtc acgag 25
<210> 23
   <211> 24
   <212> DNA
   <213> artificial sequence
<220>
   <223> forward PCR primer
<400> 23
   gtttaaagct ggcactgtcc caaa 24
<210> 24
   <211> 25
   <212> DNA
   <213> artificial sequence
<220>
   <223> reverse PCR primer
<400> 24
   ctttggcaca gtgccagctt taaac 25
<210> 25
   <211> 27
   <212> DNA
   <213> Artificial sequence
<220>
   <223> Probe
<400> 25
   ttccgccata acgacgtcaa ccatctt 27
<210> 26
   <211> 19
   <212> DNA
   <213> artificial sequence
<220>
   <223> forward PCR primer
<400> 26
   gcagggtgcc aaaacacat 19
<210> 27
   <211> 24
   <212> DNA
   <213> artificial sequence
<220>
   <223> reverse PCR primer
<400> 27
   tcgatggatc agacttcttc aaca 24
<210> 28
   <211> 24
   <212> DNA
   <213> artificial sequence
<220>
   <223> Probe
<400> 28
   tggcgtgccc attgatcaca attt 24
<210> 29
   <211> 22
   <212> DNA
   <213> artificial sequence
<220>
   <223> forward PCR primer
<400> 29
   ggtcacgctc ctggaagata gt 22
<210> 30
   <211> 20
   <212> DNA
   <213> artificial sequence
<220>
   <223> reverse PCR primer
<400> 30
   gggcactgtc aaggctgaga 20
<210> 31
   <211> 20
   <212> DNA
   <213> artificial sequence
<220>
   <223> Forward PCR primer
<400> 31
   cgggcagcag gtcgaggaga 20
<210> 32
   <211> 22
   <212> DNA
   <213> artificial sequence
<220>
   <223> reverse PCR primer
<400> 32
   caggaacggg cattttgtag ca 22
<210> 33
   <211> 27
   <212> DNA
   <213> artificial sequence
<220>
   <223> reverse PCR primer
<400> 33
   aggccaggcg aattcaacga actcggt 27
<210> 34
   <211> 27
   <212> DNA
   <213> artificial sequence
<220>
   <223> forward PCR primer
<400> 34
   cgagttcgtt gaattcgcct tggcctg 27
<210> 35
   <211> 26
   <212> DNA
   <213> artificial sequence
<220>
   <223> forward PCR primer
<400> 35
   gccacacacc tgcagaggca gtgcaa 26
<210> 36
   <211> 27
   <212> DNA
   <213> artificial sequence
<220>
   <223> forward PCR primer
<400> 36
   gcactgcctc tgcaggtgtg tggcttc 27
<210> 37
   <211> 27
   <212> DNA
   <213> artificial sequence
<220>
   <223> forward PCR primer
<400> 37
   aattcgcctc tgcaggtgtg tggcttc 27
<210> 38
   <211> 26
   <212> DNA
   <213> artificial sequence
<220>
   <223> reverse PCR primer
<400> 38
   gccacacacc tgcagaggcg aattca 26
<210> 39
   <211> 240
   <212> DNA
   <213> Homo sapiens
<400> 39
   tctggcagtc agacaggcct aagctaaacc tttccccccc agctacccac cagggtcatc 60
   cccaagccag gtcagggcca atggaggttg gggtggagaa gacaggcttg gccctatttc 120
   ctgcccaact cagaaccttc tggtttctgc cacaggatgc cttgcaagct tatcggggtc 180
   actgtgggca gctgggtgag ctaagttcat ctgtgctgcc gtgacctctg tgcagatgca 240

## Description

### Technical Field

The present invention relates to a transcriptional enhancer of the 5-aminolevulinic synthase acid gene (ALAS1) and to a method for testing chemical compounds as inducers of heme and/or P450 synthesis.

### Background Art

Humans are exposed to many foreign compounds (xenobiotics) in their diet, in their environment, and as clinically prescribed drugs (e.g., rifampicin and phenobarbital). In response to these exposures mammals have evolved mechanisms to induce proteins involved in xenobiotic detoxification. Metabolism by Phase I enzymes, particularly the heme containing monooxygenases cytochromes P450 is frequently the first line of defense against such xenobiotics. The activity of these detoxification enzymes leads to limited in vivo half lives of therapeutical drugs and consequently to a limited duration of the therapeutic effect.

Induction of drug-metabolizing enzymes by drugs and chemicals includes the transformation of drugs to inactive, active or toxic metabolites and has important clinical consequences. These consequences include drug-drug interactions and the precipitation of certain diseases such as the hepatic porphyrias. Induction of 5-aminolevulinic acid synthase by drugs, chemicals, hormones and nutrients is a hallmark of the acute attacks of hepatic porphyria, a rare inherited metabolic disease characterized by acute attacks of neuropsychiatric symptoms.

During the development of new therapeutial drugs it is therefore desirable to screen said new active compounds for their ability to induce detoxification enzymes.

The prior art describes test methods allowing a measurement of xenobiotic induction of degradation enzymes. International patent application WO 99/61622 describes a system for screening potential new drugs for susceptibility to metabolic degradation. Said method is based on a transcriptional enhancer of the human gene P450CYP3A4. Said enhancer is responsible for the transcriptional induction of the CYP3A4 gene by xenobiotic inducers including therapeutic drugs.

WO 99/48915 discloses a method of screening test compounds for their ability to induce CYP3A4 gene expression. The described method is based on the isolation of an orphan nuclear receptor designated human pregnane X receptor (hPXR) that binds e.g. to a rifampicin/dexamethasone response element in the.CYP3A4 gene regulatory region. The binding of said hPXR receptor modulates transcription of the CYP3A4 gene. The CYP3A4 enzyme is just one of 55 human CYP enzymes of which many are inducible via similar or different enhancer regions.

Although there exist already test systems allowing an evaluation of the induction of degradation enzymes by xenobiotics such as e.g. new therapeutical drugs, there is still a need for alternative means and methods allowing an easy and inexpensive testing of new therapeutical drugs for their capacity to induce any drug-metabolizing enzyme.

### Disclosure of the Invention

Hence, it is a general object of the invention to provide an isolated nucleic acid sequence which comprises at least a DR-4 nuclear receptor binding site and wherein said nucleic acid sequence functions as transcriptional enhancer of the 5-aminolevulinic acid synthase gene. Activation of said nucleic acid sequence is a marker for the induction of any cytochrome P450 gene and not just of CYP3A4.

In a preferred embodiment said nucleic acid sequence further comprises a nuclear factor 1 binding site (NF-1) and/or a DR-5 nuclear receptor binding site or has the sequence set forth in Seq. Id. No. 1.

The nucleic acid sequence of the present invention which encompasses a nuclear factor 1 binding site preferably comprises a sequence selected from the group consisting of Seq. Id. No. 2 to 7.

In a further preferred embodiment said nucleic acid sequences mediate chemical compound induced transcriptional activation. Said chemical compound is preferably a candidate compound for therapeutical use or a therapeutical drug.

Another object of the present invention is a genetic construct comprising a nucleic acid sequence of the present invention which is operably linked to a nucleic acid encoding a reporter molecule. Said reporter molecule has preferably an enzymatic activity, more preferably said reporter molecule activity can be detected by colorimetric methods, by radioactivity, fluorescence or chemiluminiscence.

Said reporter molecule is preferably selected from the group consisting of luciferase, beta-galactosidase, chloramphenicol acetyltransferase, alkaline phosphatase and green fluorescent protein.

A third object of the present invention is a method for testing compounds for modulation of heme and/or P450 cytochromes synthesis. Said method comprises the following steps: contacting suitable cells comprising a genetic construct of the present invention with a test compound and detecting enhanced/reduced expression and/or transcription of the nucleic acid sequence encoding the reporter gene. The detectable enhanced or repressed reporter gene expression and/or transcription is indicative of a compound that enhances or represses heme and/or P450 synthesis.

In a preferred embodiment said test compound is a candidate drug for therapeutical use or a therapeutical drug.

In a further preferred embodiment of the present method said enhanced expression of the nucleic acid sequence encoding the reporter gene is detected by colorimetry, fluorescence, radioactivity or chemiluminiscence.

In a particular preferred embodiment said enhanced transcription of the nucleic acid encoding the reporter gene is detected by quantitative PCR.

Preferred cells for the use in a method of the present invention are Leghorn Male Hepatoma (LMH) cells, other hepatoma cells, monkey kindney cells (CV-1, COS-1) or human kidney cells.

In a further aspect the present invention relates to the use of a nucleic acid of the present invention or a fragment thereof for the testing of chemical compounds as modulators of heme and/or P450 synthesis.

The present invention relates furthermore to the use of a genetic construct of the present invention for the testing of chemical compounds as modulators of heme and/or P450 cytochromes synthesis.

### Brief Description of the Drawings

The invention will be better understood and objects other than those set forth above will become apparent when consideration is given to the following detailed description thereof. Such description makes reference to the annexed drawings, wherein:

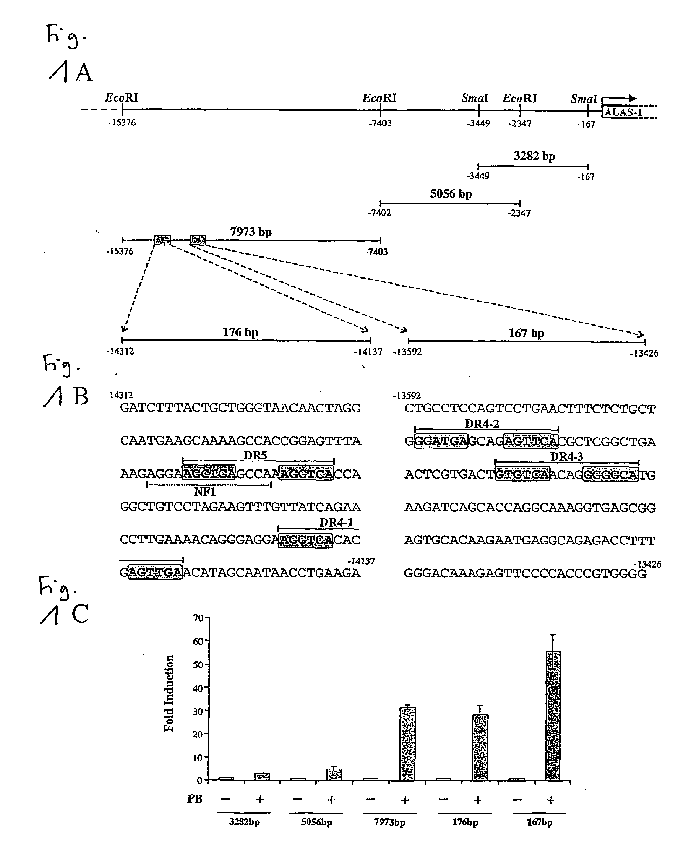

Figure 1 A shows the isolation of 176bp and 167 bp drug-responsive enhancer sequences within the first 15 kb upstream of the chicken ALAS1 transcription start site by restriction endonuclease digestion and subcloning. Fragments were cloned into the pLucMCS luciferase reporter vector containing an SV-40 promoter;
Figure 1 B shows the DNA sequences of the 176 and 167 bp enhancers. Numbering refers to sequence positions relative to the transcriptional start site of the chicken ALAS1 gene. Solid lines identify DR4 and DR5 NR binding sites. Shaded boxes contain individual half sites. A hatched line marks the NF1 binding site;
Figure 1 C shows reporter gene assays of the fragments. The constructs were transfected together with a transfection-control construct expressing β-galactosidase into LMH cells. Cells were then treated with 600 µM PB for 16 h and luciferase assays were performed on the cell extracts. Relative luciferase levels are standardized against cells transfected with vector containing no insert and expressed as fold induction. Experiments were repeated at least three times and data from a representative experiment tested in triplicate are shown here. Error bars represent standard deviations;

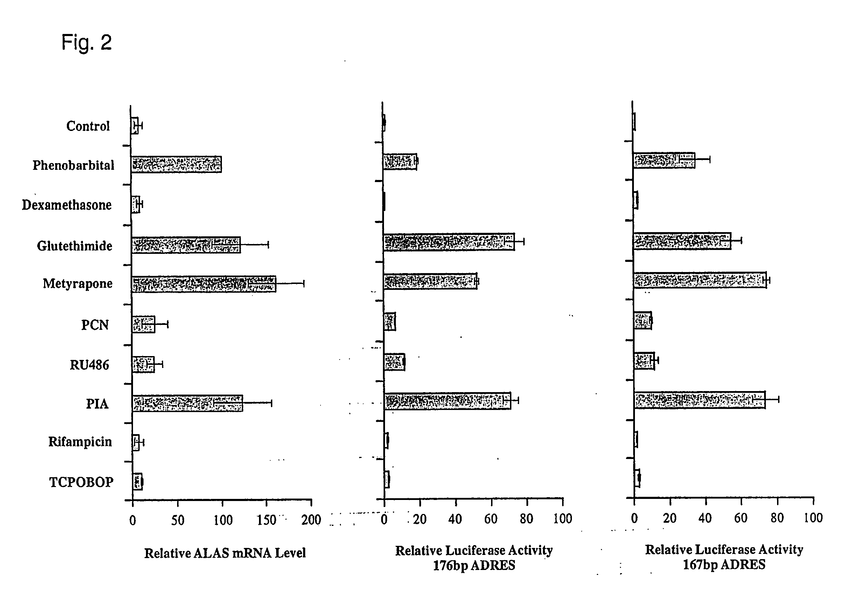

Figure 2 shows a comparison of ADRES and mRNA activation by different drugs. Relative luciferase levels are standardized against cells transfected with vector containing no insert and expressed in fold induction. Experiments were repeated at least three times and data from representative experiments tested in triplicate are shown here. Error bars represent standard deviations;

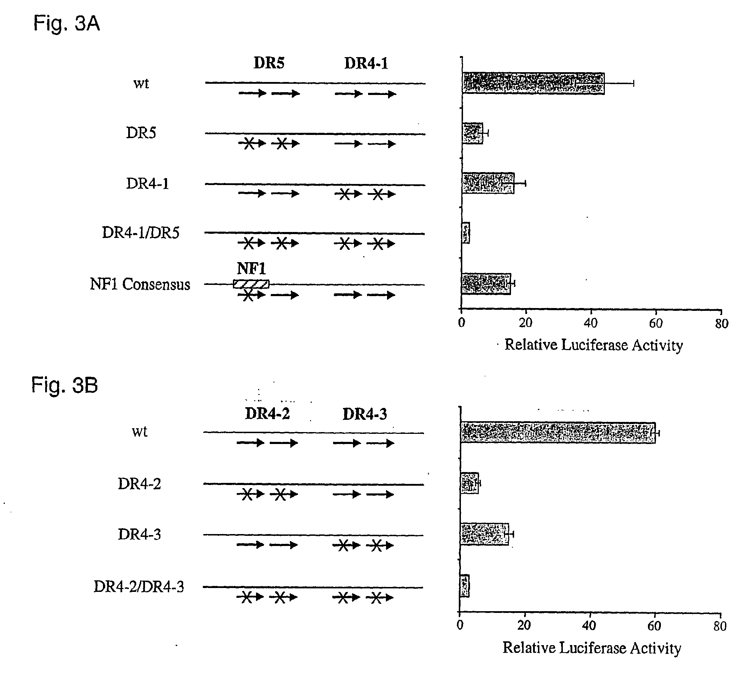

Figure 3 A shows site-directed mutagenesis of the DR4 and DR5 sites within the 176 bp ADRES element. Mutations in the DR4 and DR5 halfsites of the 176 bp sequences were introduced via PCR-based site-directed mutagenesis. Mutations are labeled in the left column and depicted with crosses in the scheme. Relative luciferase levels are standardized against cells transfected with vector containing no insert (control set to 1.0) and expressed as percentages of the 176 ADRES. Experiments were repeated at least three times and data from representative experiments tested in triplicate are shown here. Error bars represent standard deviations;
Figure 3 B shows site-directed mutagenesis of the DR4 sites within the 167 bp ADRES element. Mutations in the DR4 halfsites of the 167 bp sequence were introduced via PCR-based site-directed mutagenesis. Mutations are labeled in the left column and depicted with crosses in the scheme. The exprimental procedure was the same as described in figure 3 A;

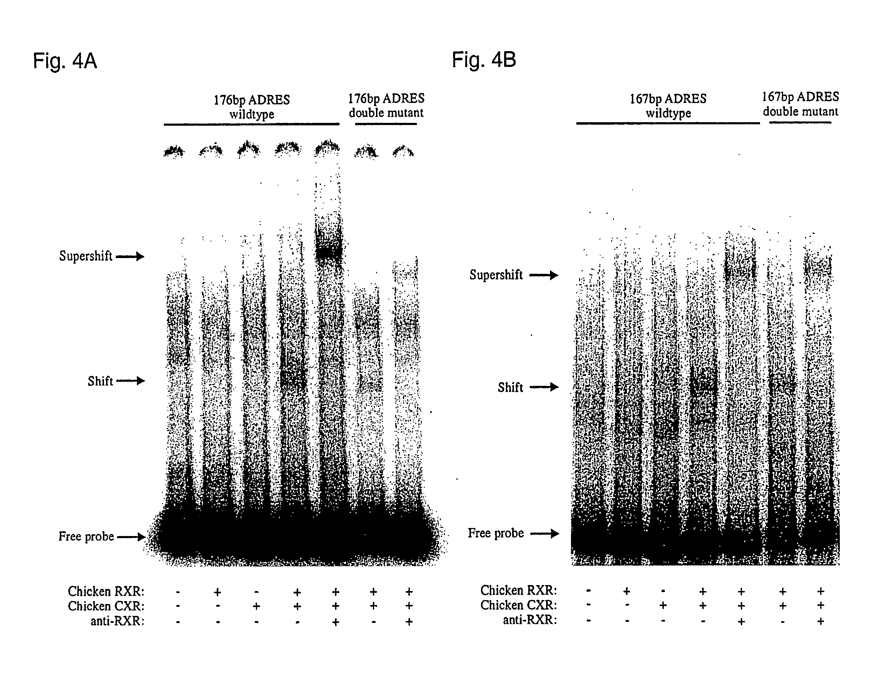

Figure 4 A shows a gel-mobility shift assay demonstrating that CXR binds the 176 bp ADRES element. Radiolabelled ADRES wild type (lanes 1-5) and mutant (lanes 6-7) sequences were incubated with *in vitro* transcribed / translated CXR (lanes 3-7), chicken RXR (lanes 2 and 4-7) and anti-RXR antibody (lane 5 and 7), as indicated. Arrows depict the unbound probe, the shifted CXR-RXR-probe complex, and the supershifted CXR-RXR-probe-anti-RXR antibody complex;
Figure 4 B shows gel-mobility shift assay demonstrating that CXR binds the 167 bp ADRES element. Radiolabelled ADRES wild type (lanes 1-5) and mutant (lanes 6-7) sequences were incubated with *in vitro* transcribed / translated CXR (lanes 3-7), chicken RXR (lanes 2 and 4-7) and anti-RXR antibody (lane 5 and 7), as indicated. Arrows depict the unbound probe, the shifted CXR-RXR-probe complex, and the supershifted CXR-RXR-probe-anti-RXR antibody complex;

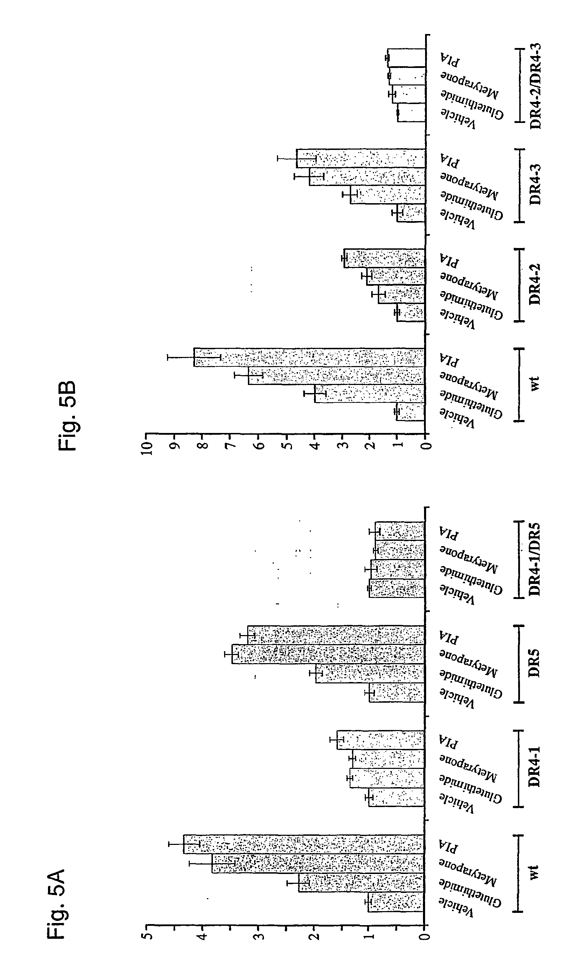

Figure 5 A shows transactivation of the 176 bp ADRES element by CXR. Cos-1 cells were transfected with constructs containing 4 repeats of the wild type, DR4-1, DR5 and DR4-1/DR5 mutants cloned into the pBLCAT5 vector containing a thymidine kinase minimal promoter. The chicken CXR coding region cloned into the pSG5 expression vector was cotransfected along with a vector expressing pSV β-galactosidase as control. Cells were then treated for 16 h with either drugs or vehicle control and extracts were analyzed for CAT expression normalized against β-galactosidase levels as described in Materials and Methods. Experiments were repeated at least three times and data from representative experiments tested in triplicate are shown here. All constructs were verified by sequencing and error bars represent standard deviations;
Figure 5 B shows transactivation of the 167 bp ADRES element by CXR. Cos-1 cells were transfected with constructs containing a single copy of the wild type, DR4-2, DR4-3 and DR4-2/DR4-3 mutants cloned into the pBLCAT5 vector containing a thymidine kinase minimal promoter. The experimental procedure was the same as described under figure 5B;

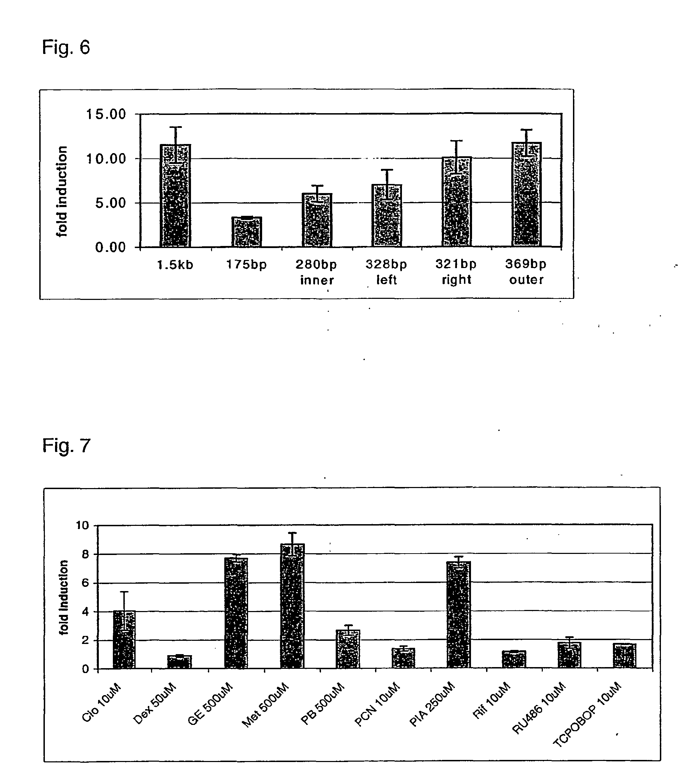

Figure 6 shows induction of expanded mouse core fragments in luciferase reporter gene assay in LMH cells. Inducer Metyrapone 500 µM;
Figure 7 shows induction of the mouse 369bp DRES by different drugs;

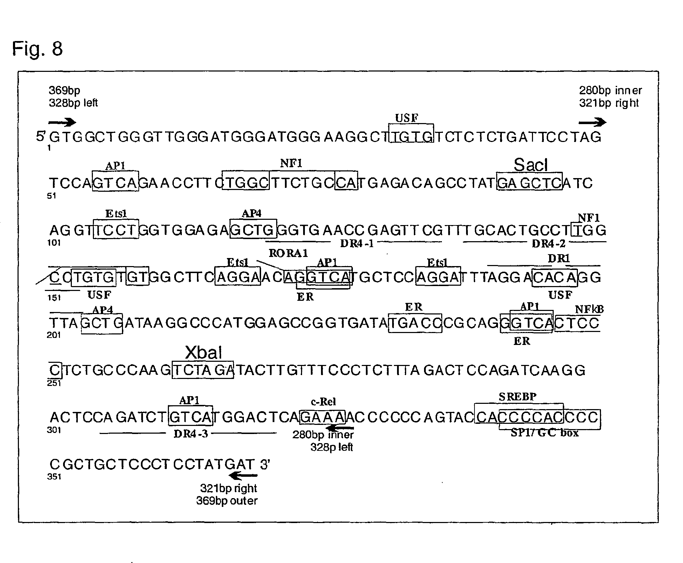

Figure 8 shows the 369bp mouse DRES sequence and discovered putative nuclear receptor binding sites;

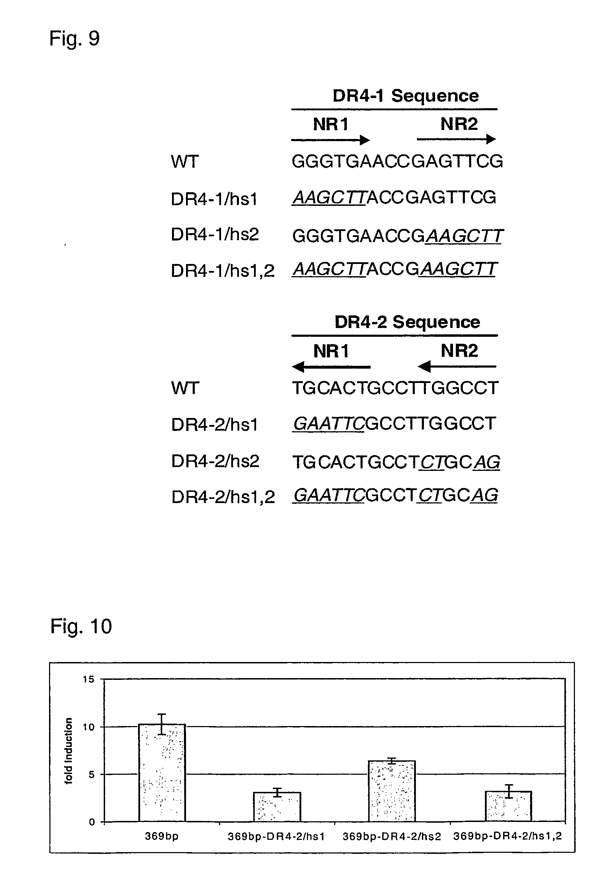

Figure 9 shows mutations introduced in the DR4-1 and DR4-2 sites of the 369bp DRES sequence. The halfsites were mutated individually and both together. Mutated base pairs are underlined and in italics;
Figure 10 shows induction of DR4 mutant constructs of mouse 369 DRES in luciferase reporter gene assay in LMH cells;

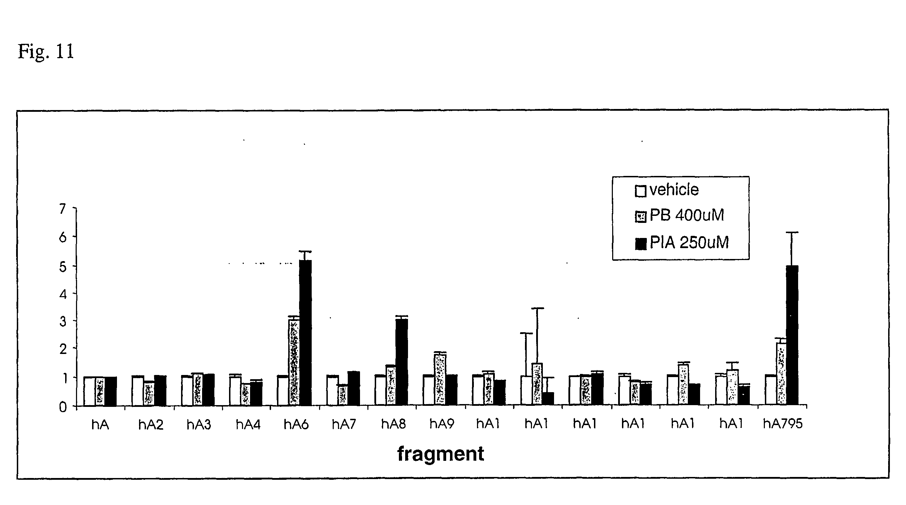

Figure 11 shows drug-induction of different fragments of the human ALAS1 gene. Fragments were cloned into the pGL3 luciferase reporter vector (Promega Corp) and tested for inducibility by the prototypical hALAS1 inducers phenobarbital (PB) and propylisopropylacetamid (PIA). Four hours after transfection of LMH cells with the constructs, cells were exposed to the drugs for 24 hours, after which luciferase activity was assayed. Results were normalized for transfection efficiency by assaying for activity of co-transfected β-galactosidase. Data shown is one representative experiment;

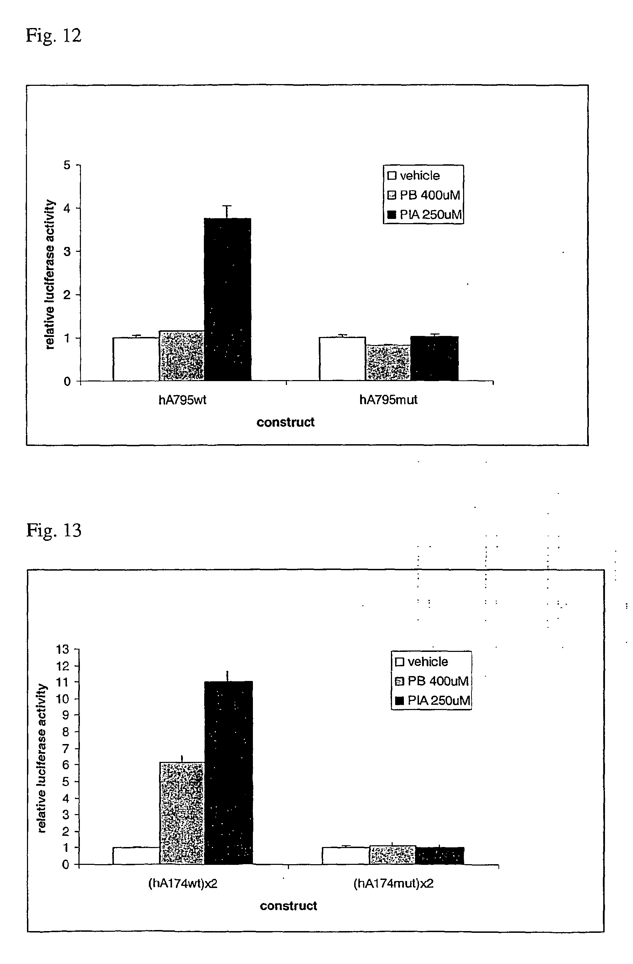

Figure 12 shows that the effect of drugs on the hA795 element depends on the presence of a DR-4 motif. Within the sequence of the hA795 fragment, a putative DR-4 type nuclear receptor response element was found by computer analysis. Site-directed mutagenesis of this element abolished inducibility of this fragment in reporter gene assays in LMH cells. Experiments were performed as described under figure 8;
Figure 13 shows that a core sequence spanning the DR-4 element is sufficient to mediate drug induction in LMH cells. From the hA795 fragment, the hA174 fragment was derived. It is 174bp in length and within its sequence, the DR-4 response element is contained. Direct repeats of the wildtype hA174 or a mutant, where the DR-4 was destroyed were cloned into the pGL3 reporter vector and tested in LMH cells;

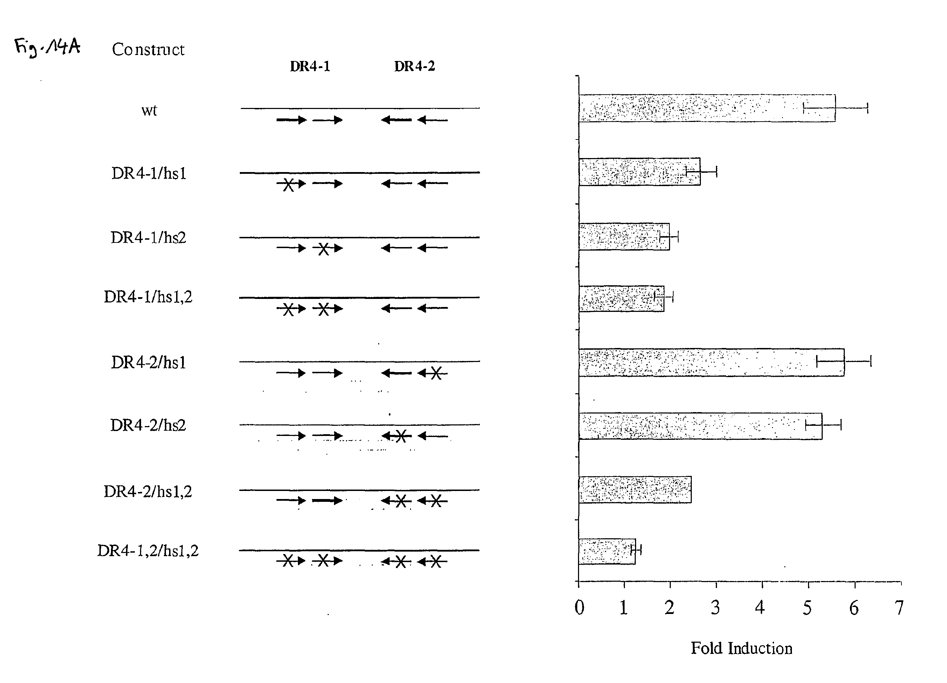

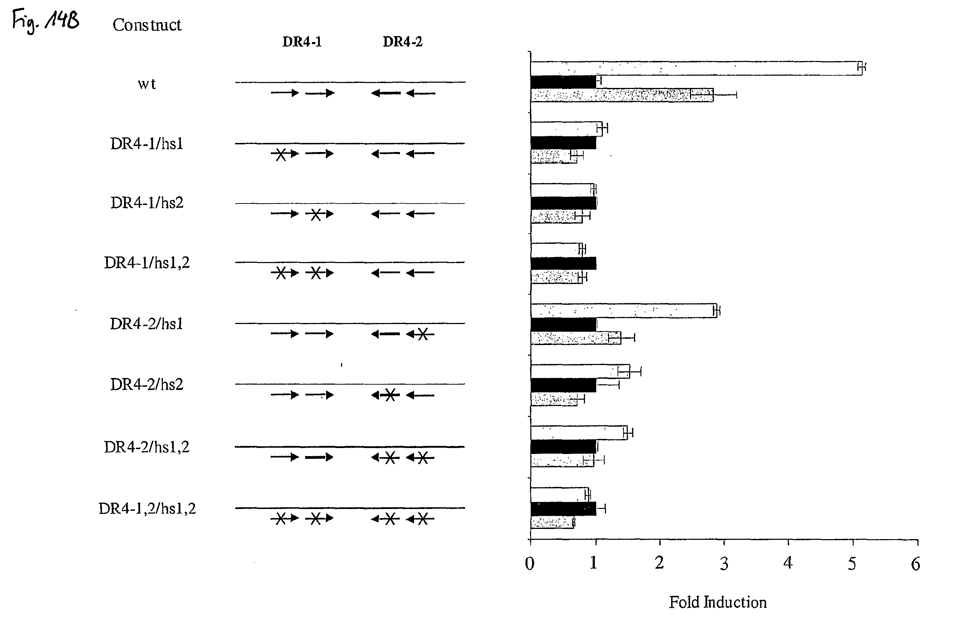

Figures 14A and B show that the ALA synthase drug responsive enhancer sequence is inducible by mouse PXR (14A) and mouse CAR (14B) in transactivation assays;

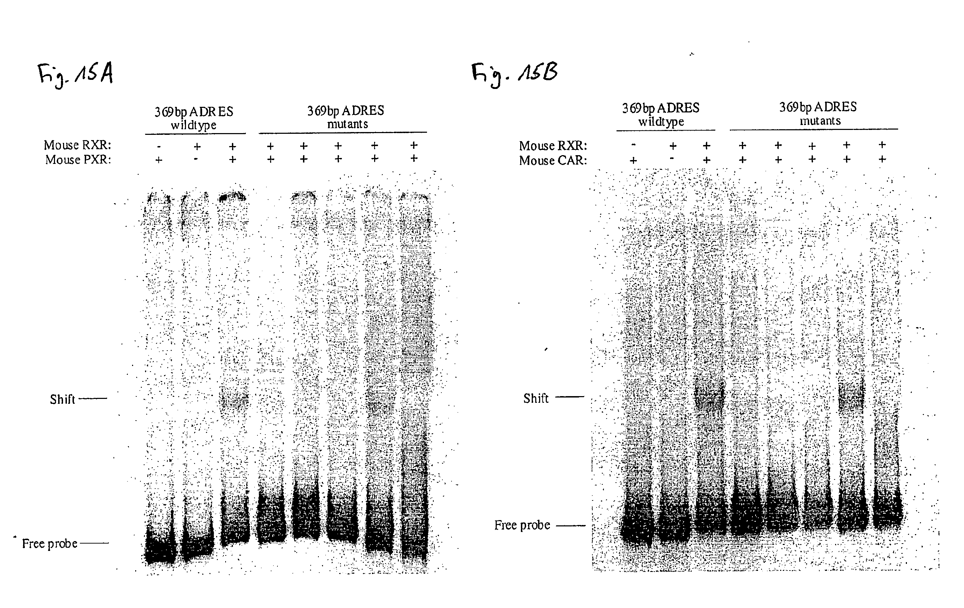

Figures 15A and B show the results of gel shift assays of 369bp ADRES with PXR (15A) and CAR (15B)';

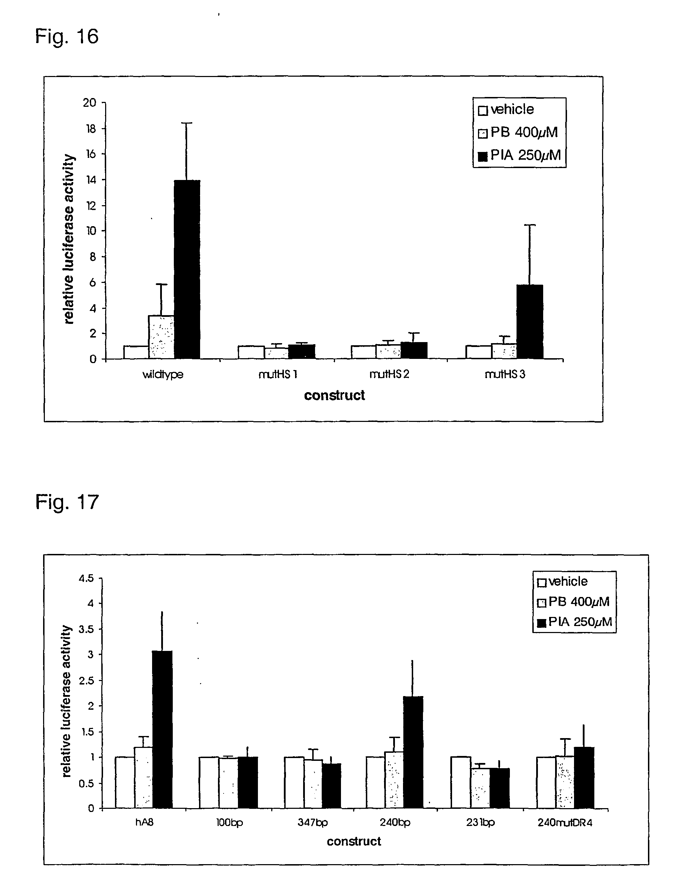

Figure 16 shows the hA174bp core element derived from the hA795 fragment conferring drug-mediated transcriptional activation to a reporter gene in LMH cells;

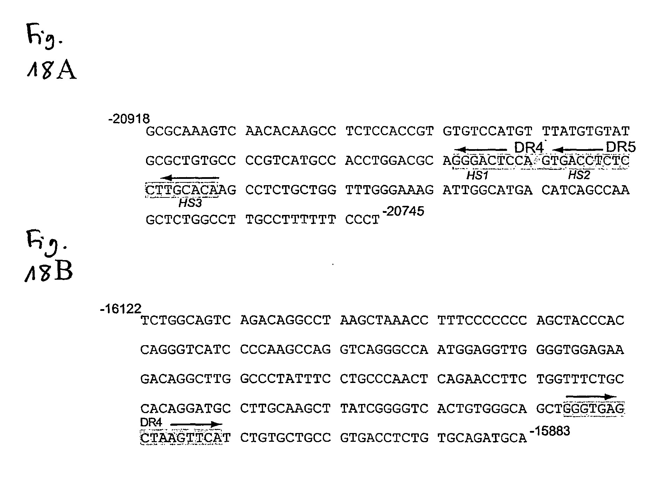

Figure 17 shows the result of a drug induction assay in LMH cells using sub-fragments of the hA8 drug responsive element;

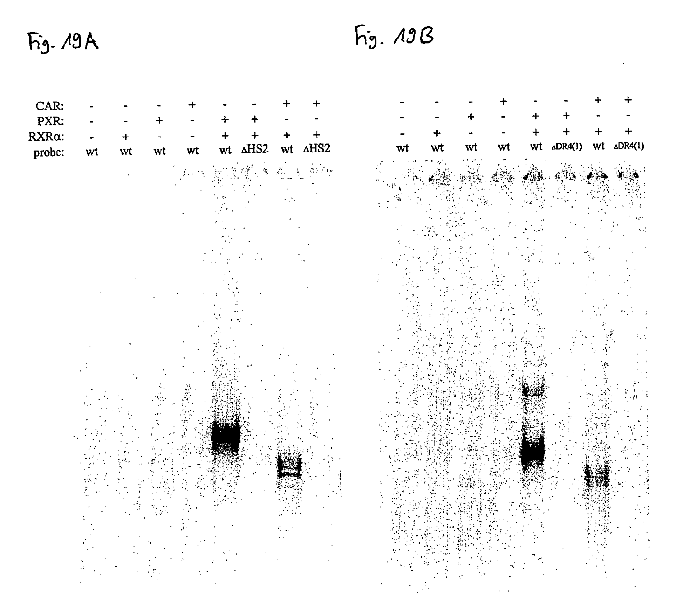

Figure 18A depicts the hA174bp core element of the drug responsive element within the human ALAS1 gene;
Figure 18B depicts the hA240bp core element of the drug responsive element within the human ALAS1 gene;
Figures 19 A and B depict the results of an electrophoretic mobility shift assay, assaying the ability of human PXR and human CAR to bind to the hA174bp and hA240bp elements.

### Modes for Carrying Out the Invention

Heme is an essential component in oxygen transport and metabolism in living systems. In nonerythropoietic cells, the first and rate-limiting enzyme in the pathway, 5-aminolevulinic acid synthase (ALAS1), regulates its biosynthesis. Under normal physiological conditions, free heme levels are low and tightly regulated, as toxicity can occur with increased cellular concentrations of unincorporated heme. Following administration of drugs such as phenobarbital (PB) or other prototypical CYP inducers, heme concentrations are elevated in the liver to accommodate the increased levels of heme dependent enzymes. This is achieved by induction of ALAS1 and assures an adequate and apparently coordinated supply of heme for the generation of functional cytochrome holo-proteins such as e.g. cytochromes P450 (CYP).

In the scope of the present invention the inventors have identified and characterised nucleic acid elements in the 5' flanking region of the gene encoding ALAS1 which functions as an enhancer for ALAS1 gene transcription. Said identified nucleic acid element is responsible for chemical compound induced ALAS1 gene transcription.

Said nucleic acid elements comprise at least a DR-4 nuclear receptor binding site. The term "DR-4 nuclear receptor binding site" as used herein refers to a direct repeat-4 hexamer repeat. Such a binding site is characterised by hexamer half sites arranged as direct repeats with a 4 nucleotide separation between half-sites. The half-site has the following canonical sequence AG(T/G)TCA. The term as used herein comprises as well functional equivalents of the canonical sequence i.e. half-site sequence variants which are still able to function as binding sites for nuclear receptors such as e.g. CAR (constitutive androstane receptor)/RXR (retinoid X receptor) heterodimers.

In a preferred embodiment said ALAS1 gene enhancer further comprises a NF-1 binding site. The term "NF-1 binding site" as used herein refers to a DNA element which serves as binding site for members of the nuclear factor-1 family of transcription factors, and said term encompasses functional equivalents thereof i.e. sequence variants which are still able to function as binding sites for members of the nuclear factor 1 (NF1) family of transcription factors. The NF-1 binding site has the following consensus sequence: TGGC(N₄)GCCA (N= any nucleotide). For a man skilled in the art it is clear that the sequences of the present invention can comprise more than one copy of the above identified binding sites.

In the scope of the present invention the following sequences conferring chemical compound induced ALAS1 gene transcription were characterised: Seq. Id. No. 1 (chicken), Seq. Id. No. 2 (chicken), Seq. Id. No. 3 to 7 (mus musculus), Seq. Id. No. 8 to 10 and Seq. Id. No. 39 (homo sapiens). The nucleic acid sequences set ,forth in Seq. Id. No. 8 to 10 and 39 have been part of a data-bank before the filing date of this application but these sequences have not been characterised and their function/activity has been unknown.

It has to be understood that the term nucleic acid sequence as used herein encompasses fragments, variants or derivatives of the sequences 1 to 10 of the present invention. Based on the disclosed enhancer sequences of ALAS1 and well known molecular biological methods as e.g. described in Sambrook et al., Molecular Cloning: A Laboratory Manual, New York: Cold Spring Harbor Laboratory, 2001) a man skilled in the art is able to isolate further sequences conferring chemical compound induced ALAS1 gene transcription/expression.

The construction of a genetic construct of the present invention can be done using standard molecular biology techniques as described e.g. in Sambrook et al., Molecular Cloning: A Laboratory Manual, New York: Cold Spring Harbor Laboratory, 2001).

The cell used in a test method can be any suitable cell allowing the performance of chemical compound induced assays. A particularly preferred cell line for the use in a method of the present invention is the Leghorn male hepatoma (LMH) cell line. The genetic construct can be introduced in the cells by well known transfection methods such as e.g. chemical transfection, elektrotransfection or viral transfection. Said host cell can express the genetic construct of the invention from a genomic locus or from an expression vector. Typically, an expression vector comprises the regulatory sequences required to achieve transcription and expression in the host cell and it may contain necessary sequences required for plasmid replication in order to exist in an episomal state, or it may be designed for chromosomal integration. A suitable vector is e.g. pGL3LUCpro (Promega) which comprises the gene encoding luciferase.

The method for testing compounds for modulation of heme and/or P450 synthesis of the present invention comprises the following steps: suitable cells harboring a genetic construct of the present invention are contacted with a test compound and an enhanced or repressed reporter gene expression and/or transcription is detected. The detection method of the enhanced or repressed gene transcription/expression is depending on the used reporter gene and can be done at the transcriptional level using e.g. quantitative PCR or by detecting the reporter gene product. Preferred detection methods for the reporter gene product are colorimetric, fluorescence or chemiluminiscence assays such as e.g. luciferase assay, CAT assay or β-galactosidase assay.

The invention is now further described by means of examples:

### Isolation and characterization of a drug responsive enhancer of the chicken 5-aminolevulinic acid gene

A cosmid clone containing an insert approximately 35 kb in length spanning the chicken ALAS1 gene and 15 kb of the 5'-flanking region, was isolated and its sequence analyzed. Three major subclones were generated from the region upstream of the transcriptional start site, including a 3282 bp *SmaI* fragment and 5056 bp and 7973 bp *EcoRI* segments (Fig. 1A). The *SmaI* clone extends from -167 bp to -3449 bp, whereas the *EcoRI* subfragments span the regions from -2347 bp to -7402 bp and -7403 bp to -15376 bp, respectively. These subfragments were cloned into the pLucMCS modified luciferase vector containing an SV40 promoter as described in Materials and Methods. Drug inducibility was measured in transiently transfected LMH cells treated with 600 µM PB and compared with control values. The results revealed the 7973 bp subfragment to be highly inducible with PB, displaying a 28-fold increase in transcriptional activation relative to control values. In comparison, the 5056 bp and 3282 bp subfragments exhibited virtually no transcriptional activation in response to drug treatment (Fig. 1C). The 7973 bp subfragment (-15376 / -7403) was chosen for further analysis and was divided into numerous subclones in the pLucMCS reporter vector resulting in the isolation of 176 bp *Sau3AI-SmaI* and 167 bp *PvuII-HaeIII* elements (Fig. 1A and 1B). These sequences routinely exhibit 25-60 fold induction over control values in reporter gene assays when exposed to PB in LMH cells (Fig. 1C). All other portions of the 7973 bp fragment were also subcloned but displayed no drug response when tested in LMH cells. Because the 176 bp (Seq. Id. No. 2) and 167 bp (Seq. Id. No. 1) fragments retain high drug response regardless of orientation or distance from the promoter they are referred to as aminolevulinic acid synthase drug responsive enhancer sequence (ADRES) elements.

Recent discoveries have implicated NRs in drug mediated enzyme induction (2, 3, 8). For this reason, we scanned the responsive elements for potential nuclear receptor response sites using a computer algorithm based on a weighted nucleotide distribution matrix compiled from published functional hexamer halfsites. Two potential binding sites for orphan NRs were identified in each ADRES element, having two direct repeats with 4 nucleotide (DR4) and 5 nucleotide (DR5) separations between halfsites in the 176 bp sequence and two direct repeats with 4 nucleotide (DR4) separations between halfsites in the 167 bp sequence (Fig. 1B). For clarity, the three DR4 binding sites are labeled according to their occurrence in the gene, with the furthest upstream from the transcription start site called DR4-1 and the closest to the start site DR4-3. The putative DR4-1 is defined by one perfect half-site (AGGTCA) and one imperfect half-site (AGTTGA) at -14186/-14181 and -14176/-14171 respectively, whereas the DR5 site is characterized by an imperfect upstream half-site (AGCTGA) and a perfect downstream half-site (AGGTCA) at -14251/-14246 and -14240/-14235. In the 167 bp sequence, DR4-2 consists of one imperfect upstream half-site (GGATGA) and one perfect downstream half-site (AGTTCA) at -13563/-13558 and -13553/-13548 and DR4-3 has two imperfect halfsites (GTGTCA and GGGGCA) at -13526/-13521 and -13516/-13511. It is interesting to note that the 176 bp ADRES also contains a putative binding site for nuclear factor 1 which overlaps the DR5, spanning bp -14255 to bp -14242, whereas the 167 bp ADRES does not.

We next wanted to compare ADRES-mediated ALAS1 induction levels from reporter gene assays with stimulation of transcription in a physiological system. Therefore, ALAS1 mRNA levels were quantified in LMH cells cultured in serum-free medium and 16 h of exposure to a variety of chemical inducers and compared to the induction pattern observed with the same compounds in transient transfections of the ADRES (Fig. 2). The compounds examined include PB (600 µM) and the PB-like inducers PIA (250 µM), glutethimide (500 µM), and the potent mouse CYP 2B inducer 1,4-bis[2-(3,5-dichloropyridyl-oxy)]benzene (TCPOBOP) (10 µM). In addition, the common CYP3A inducers dexamethasone (50 µM), metyrapone (400 µM), and 10 µM mifepristone (RU-486) were employed for comparison. We were also interested in the effects of 10 µM 5-pregnen-3β-ol-20-one-16α-carbonitrile (PCN) and rifampicin (100 µM) due to their species-specific effects on PXR activation and CYP3A induction. Messenger RNA was reverse transcribed and levels of ALAS1 cDNA were quantified using the Taqman real-time PCR quantification system as described in Materials and Methods. PB was a strong inducer of ALAS1 in LMH cells, increasing RNA levels an average of 16 fold relative to basal transcript levels (Fig. 2). This value was chosen to represent 100% induction, against which all other values are compared. The general inducers PIA and glutethimide, as well as the 3A-specific inducer metyrapone exhibited the strongest effects upon the ADRES elements, stimulating transcription in excess of levels obtained from PB treatment. In comparison, dexamethasone, PCN, RU-486, and rifampicin had minor or no effects on either mRNA levels or ADRES activation. Moreover, the mouse-specific compound TCPOBOP elicited no response in either mRNA transcription or stimulation of the ADRES in reporter assays. When comparing the induction profiles of the two ADRES elements to each other, very few differences are in evidence. The 167 bp (Seq. Id. No. 1) responds to PB with twice the activation when compared to the 176 bp element (Seq. Id. No. 2). Also, the 176 bp has slightly more affinity for glutethimide than metyrapone, whereas the 167 bp element exhibits a stronger response to metyrapone than glutethimide. These experiments indicate a high degree of similarity in the relative activation of the ADRES elements in reporter gene assays to each other and to mRNA transcript levels from chemically induced LMH cells.

Site-specific mutagenesis was used to examine the roles of specific nucleotides within the putative DR5 and DR4 recognition sequences in conferring drug response to the ADRES elements (Fig. 3). Mutant constructs of the DR4 and DR5 core recognition sites destroying the putative NR binding sites were generated as described in Materials and Methods. Briefly, primers were used in conjunction with PCR to convert the 5' and 3' half-sites of the DR5 to *Eco*RI and *Pst*I restriction endonuclease sites, respectively. Similarly, the DR4-3 half-sites were converted to *Eco*RI and *Nco*I restriction endonuclease sites. Data from a nucleotide distribution matrix for halfsites developed by M. Podvinec in this laboratory was applied to ascertain that the mutated halfsites least resemble functional halfsites. DR4-1 halfsites were obliterated by converting AGGTCA and AGTTGA halfsites to unconserved ACTCGA and ATACCA bases, respectively. Similarly, DR4-2 halfsites were both converted from GGATGA and AGTTCA nucleotides to CCCCAC bases. Primers were used to generate constructs mutated at each individual and both NR binding sites within both of the ADRES elements as shown in figure 3.

The modified enhancers were examined for response to 600 µM PB in luciferase reporter gene assays and the results are presented in Figure 3. These findings indicate that both the DR5 and DR4 recognition sites in the 176 bp ADRES and both DR4 recognition sites in the 167 bp ADRES element are essential to elicit full drug response. Mutation of the DR5 reduced activity of the 176 bp ADRES element by over 85% from 44 fold to 6.4 fold activation by PB, whereas changes in the DR4-1 limited activation by over 60% from 44 fold to 16 fold stimulation (Fig. 3A). As depicted in Figure 3B, both DR4-2 and DR4-3 sites in the 167 bp ADRES element were found to be required for full activation by PB. Alteration of the DR4-2 site resulted in the reduction of PB response by over 90% from 60 fold to 5.4 fold. Mutations in the DR4-3 site caused PB response to be diminished 75% from 60 to 15 fold induction. These studies demonstrate an essential contribution of the sequences within the putative DR4 and DR5 NR binding sites to PB activation of the ADRES elements.

Because the DR5 overlaps a putative binding site of NF1, a transcription factor that has been implicated in modifying drug induction, we tested the possibility that NF1 confers activation to the 176 bp ADRES element rather than NRs binding to the DR5 (5). A mutant construct converting the putative NF1 site to a consensus avian NF1 binding site, thus destroying the first half-site of the DR5, was generated and tested in luciferase assays. As seen in Figure 3A, the NF1 consensus sequence does not increase the response of the 176 bp ADRES element to drugs. Rather, the induction is decreased by 66 percent from 44 to 15 fold induction, presumably due to the destruction of the DR5 NR binding site. In order to confirm these findings, chicken NF1-A was amplified from a cDNA library generated from LMH cells and cloned into pSG5 expression vector. Coding sequence fidelity was confirmed by sequencing and the construct was co-transfected both in induction experiments in LMH cells and transactivations in Cos-1 cells, resulting in no changes in induction or transactivation.

Recent findings have implicated a number of orphan NRs in drug induction of cytochromes P450 (for reviews, see (6, 10, 11). Our group has successfully cloned and expressed chicken CXR and has demonstrated CXR-RXR interactions with CYP enhancers in electrophoretic mobility shift assays (EMSAs) (2). As the DR4 and DR5 sites clearly contribute to the transcriptional activation exhibited by the ADRES elements, gel-mobility shift assays were used to determine whether CXR might bind the responsive enhancers (Fig. 4). Neither in vitro transcribed/translated chicken CXR nor chicken RXR alone bound to the ³²P-radiolabeled 176 bp ADRES (Fig. 4A, lanes 2 and 3) or to the 167 bp ADRES (Fig. 4B, lanes 2 and 3). In contrast, CXR/RXR heterodimers bind the drug responsive enhancers, and these complexes could be supershifted with anti-RXR antibodies (Fig. 4A and 4B', lanes 4 and 5). Nuclear receptor binding to the 167 bp ADRES element was reduced when the double mutant DNA sequences were used, as demonstrated by the reduced band intensities of the shifted and supershifted components (Fig. 4B, lanes 6 and 7). Moreover, the binding of CXR/RXR heterodimers was virtually eliminated when both binding sites in the 176 bp ADRES elements were mutated (Fig. 4A, lanes 6 and 7). These findings demonstrate interactions of CXR/RXR heterodimers with the ADRES elements through the DR4 and DR5 NR binding sites.

In order to confirm the role of CXR in the activation of the ADRES elements, transactivation experiments were done in Cos-1 monkey kidney cells. These cells express RXR but exhibit no induction response under normal conditions. Four copies of the wild type and mutated 176 bp element or a single copy of the mutated and wild type 167 bp element were cloned into the pBLCAT5 plasmid containing a tk minimal promoter as described in Materials and Methods. CAT vectors were used for transactivations rather than luciferase because CAT provided more stable expression and showed higher drug response. These constructs were cotransfected along with a pSG5 expression vector containing the coding sequence for CXR and a β-galactosidase expression construct to correct for variations in transfection efficiency. After 24 h incubation to allow for the expression of CXR, induction of the wild type and mutant sequences was tested with glutethimide, metyrapone and PIA, the three best inducers identified in figure 2. As shown in Figure 5, both the 176 bp and 167 bp ADRES elements are transactivated by CXR. n the 176 bp element, the induction was reduced by 10-25% in the constructs carrying the mutant DR5 NR binding site. The mutations in the DR4-1 binding site reduced the induction by all drugs to less than 1.6 fold. Moreover, the alteration of both NR binding sites in the 176 bp element resulted in the complete elimination of drug response. The 167 bp element was found to respond better to drugs in transactivatons than the 176 bp element, thus a single copy was sufficient. The induction of the wild type sequence was strong for all three drugs, ranging from 4.0 to 8.3 fold over uninduced levels (Fig. 5B). The DR4-2 mutants exhibited lower induction after drug exposure, reduced by 58-66% when compared to wild type values. Similarly, the DR4-3 mutant sequences responded to drugs with diminished capacity, exhibiting 55-66% of the 167 bp activity. The double mutant 167 bp element did not respond to drugs, confirming the role of CXR in activating the ADRES elements via the DR4 and DR5 NR binding sites.

### Material and Methods

### Reagents

Dexamethasone, metyrapone (2-methyl-1,2-di-3-pyridyl-propadone), 5-pregnene-3β-ol-20-one-16α-carbonitrile (PCN) and rifampicin were purchased from Sigma chemical company. Propylisopropylacetamide (PIA) was a gift from Dr. Peter Sinclair (Veterans Affairs Hospital, White River Junction, VT). Glutethimide was purchased from Aldrich. Mifepristone (RU-486) was obtained from Roussel-UCLAF. 1,4-Bis[2-(3,5-dichloropyridyloxy)]benzene (TCPOBOP) was generously provided by U. Schmidt (Institute of Toxicology, Bayer, Wuppertal, Germany). Phenobarbital sodium salt (5-ethyl-5-phenyl-barbituric acid sodium salt) was purchased from Fluka. Tissue culture reagents, media, and sera were purchased from Life Technologies. All other reagents and supplies were obtained from standard sources.

### Plasmids

The pGL3LUC luciferase reporter containing an SV40 promoter was purchased from Promega. The reporter plasmid was modified by the addition of the fragment spanning the *Sac*I to the *Xho*I restriction endonuclease sites of the multiple cloning site of the pBluescript SK vector (Stratagene) to the pGL3LUC vector, thus greatly enhancing the cloning versatility of the new pLucMCS reporter. The pBLCAT5 chloramphenicol acetyl transferase reporter vector was described previously (1). Chicken CXR and RXR were cloned into the pSG5 expression vector (Stratagene) as previously reported (2). The pRSV β-galactosidase vector used for normalization of transfection experiments was kindly provided by Anastasia Kralli (Biozentrum, University of Basel, Basel, Switzerland).

### Cosmid Isolation

A specific probe for the ALAS1 gene was generated via PCR using chicken embryo liver genomic DNA as template and forward primer 5'-CGG GCA GCA GGT CGA GGA GA-3' (Seq. Id. No. 31) and reverse primer 5'-CAG GAA CGG GCA TTT TGT AGC A-3'(Seq. Id. No. 32). The probe was ³²P-radiolabeled using the random primer labeling kit (Roche Molecular Biochemicals) according to the manufacturer's instructions. A genomic cosmid library generated from adult male Leghorn chicken liver was purchased from Clontech Laboratories. The ALAS1 probe was used to identify an individual cosmid clone containing the ALAS1 gene and at least 15 kb of 5'-flanking region was isolated and confirmed by sequencing.

### Construction of vectors

The cosmid containing the ALAS1 gene and flanking region was digested with *Eco*RI restriction endonuclease and subfragments of the approximately 35 kb of new sequence were cloned into the *Eco*RI site of the pLucMCS vector. Eight fragments ranging in size from 10 kb to 900 bp in length were cloned. In addition, a 3282 bp *Sma*I fragment encoding the ALAS1 promoter region and proximal 5'- flanking region was cloned into pLucMCS. The drug-responsive 8 kb *Eco*RI region was then further subdivided using standard subcloning procedures and restriction endonucleases to isolate the *Sau*3AI-*Sma*I 176 bp element and the *Pvu*II-*Hae*III 167 bp element. Single copies of the 176 bp and 167 bp wild type and mutated elements were cloned into pBLCAT5 by excising a 222 bp fragment containing the desired sequences with *Bam*HI and *Bgl*II restriction endonucleases and ligating them into *Bam*HI-linearized pBLCAT5 vector. Multiple repeats of the 176 bp wild type and mutant elements were subcloned by inserting the 222 bp fragment 4 times in succession into the *Bam*HI-linearized pBLCAT5 vector.

### Cell Culture

Leghorn Male Hepatoma (LMH) cells were obtained from the American Type Culture Collection and cultivated in 10 cm dishes in Williams E medium supplemented with 10% FCS, 1% glutamine (2mM) and 1% penicillin/streptomycin (50 IU/ml). Dishes coated with 0.1% gelatin were used for routine culture of LMH cells in order to facilitate proper seating of the cells onto the plastic plate surface. For transfections, cells were seeded onto 12-well Falcon 3043 dishes and expanded to 70-80% surface density. Cells were then maintained in serum-free Williams E media for 24 hours and transfected using the FuGENE 6 transfection reagent (Roche Molecular Biochemicals) according to the manufacturers protocol.

### Analysis of Reporter Gene Expression

Cells were treated with drugs or vehicle for 16 h and harvested. For luciferase assays, lysis was performed with 200 µl Passive Lysis Buffer (Promega) per well and extracts were centrifuged for 1 minute to pellet cellular debris. Luciferase assays were performed on supernatants using the Luciferase Assay kit (Promega) and a Microlite TLX1 luminometer (Dynatech). Relative β-galactosidase activities were determined as described (4). For CAT assays, cells were lysed with 600 µl CAT lysis buffer per well and extracts were centrifuged for 1 minute to pellet cellular debris. Assays were performed using a CAT ELISA kit (Roche Molecular Biochemicals) according to the manufacturers protocol.

### Site-directed Mutagenesis

Mutations in the putative NR binding sites were introduced into the ADRES elements by PCR using standard overlap techniques. Briefly, subfragments were amplified with overlapping primers carrying the desired mutations and vector primers. These subfragments were then combined and used as template in a second PCR using vector primers to amplify the full-length mutated fragment, which was subsequently digested with appropriate enzymes and cloned into pLucMCS. The forward vector primer was the RV primer 3 and the reverse vector primer was the GL primer 2 within the pGL3 luciferase vector (Promega). All mutations are shown in bold. DR4-1 double mutation constructs were generated with 5'-GGA GGA **ACT CG**A CAC GA**T ACC** AAC ATA GCA AT-3' forward (Seq. Id. No. 15) and 5'-CTA TGT T**GG TA**T CGT GT**C GAG** TTC CTC CCT G-3' reverse (Seq. Id. No. 16) primers. DR5 double mutants were amplified with 5'-**GAA TTC** GCC AAC **TGC AG**C CAG GCT GTC C-3' forward (Seq. Id. No. 17) and 5'-CAG CCT GGC **TGC AGT** TGG C**GA ATT C**TC CTC-3' reverse (Seq. Id. No. 18) primers. DR4-2 double mutants were generated with 5'**-CCC CAC** GCA G**CC CCA C**CG CTC GGC TGA ACT CGT G-3' forward (Seq. Id. No. 19) and 5'-**GTG GGG** CTG C**GT GGG G**CA GCA GAG AAA GTT CAG G -3' reverse (Seq. Id. No. 20) primers. DR4-3 double mutants were amplified using a 5'-**GAA TTC** ACA G**CC ATG G**TG AAG ATC AGC-3' forward (Seq. Id. No. 21) primer and a 5'-**CCA TGG** CTG **TGA ATT** CAG TCA CGA G-3' reverse (Seq. Id. No. 22) primer. Avian NF1 consensus sequence was generated using 5'-GTT TAA AG**C T**GG **CA**C TG**T** CCC AAA-3' (Seq. Id. No. 23) and 5'-CTT TGG C**A**C AG**T GC**C **AG**C TTT AAA C-3' (Seq. Id. No. 24) forward and reverse primers (9). Following PCR overlap, the products were digested with *Bgl*II and either *Eco*RI or *Not*I restriction endonucleases and cloned into pLucMCS. All constructs were verified by sequencing.

### Quantitative PCR

LMH cells were plated onto 12-well plates, expanded to 70-80% surface density and incubated in serum-free media for 24 h. Cells were then exposed to either drug or vehicle and RNA was isolated with Trizol reagent (Gibco BRL) according to the manufacturer protocol. One µg of total RNA was reverse transcribed with the Moloney murine leukemia virus reverse transcriptase kit (Roche Molecular Biochemicals). PCR was performed using the Taqman PCR core reagent kit (PE Applied Biosystems) and transcript levels quantitated with an ABI Prism 7700 sequence detection system (PE Applied Biosystems). Relative transcript levels were determined using the relative quantitation method measuring the ΔΔCt. The following primers and probes were used in these reactions. ALAS1: probe, 5'-TTC CGC CAT AAC GAC GTC AAC CAT CTT-3'(Seq. Id. No. 25) ; forward primer, 5'-GCA GGG TGC CAA AAC ACA T-3' (Seq. Id. No. 26); reverse primer, 5'-TCG ATG GAT CAG ACT TCT TCA ACA-3'(Seq. Id. No. 27). Glyceraldehyde-3-phosphate dehydrogenase (GAPDH): probe, 5'-TGG CGT GCC CAT TGA TCA CAA TTT-3' (Seq. Id. No. 28); forward primer, 5'-GGT CAC GCT CCT GGA AGA TAG T-3' (Seq. Id. No. 29); reverse primer, 5'-GGG CAC TGT CAA GGC TGA GA-3' (Seq. Id. No. 30). Transcript levels were measured in separate tubes and GAPDH values were used for normalization of ALAS1 values.

### Gel mobility-shift assays

Chicken CXR and RXR proteins were expressed using the TNT T7 Quick Coupled Translation System (Promega) according to the manufacturers protocol. Probes were labeled by Klenow reaction in the presence of radiolabeled [α-³²P]ATP and purified over a Biospin 6 chromatography column. A volume of labeled oligonucleotide corresponding to 100,000 cpm was used for each reaction in 10 mM Tris-HCL (8.0) / 40 mM KCl / 0.05% Nonidet P40 / 6% glycerol (vol./vol.) / 1 mM DTT containing 0.2 µg of poly(dI-dC) and 2.5 µl *in-vitro* synthesized proteins as described previously (2, 7). To test for supershifts, 0.5 µl monoclonal anti-mouse-RXR rabbit antibody (kindly provided by P. Chambon, Université Louis Pasteur, Illkirch, France) were added to the reaction mix. This antibody has been previously tested for interactions with chicken RXR in Western blots (data not shown). The reaction mix was incubated for 20 min at room temperature and electrophoresed on a 6% polyacrylamide gel in 0.5X Tris / borate /EDTA buffer followed by autoradiography.

### Transactivations

Experiments to determine the contribution of the nuclear receptor CXR to the induction of ALAS-1 were tested in Cos-1 monkey kidney cells (generously provided by A. Kralli, Biozentrum, University of Basel, Basel, Switzerland) according to methods previously described (2). Briefly, cells were expanded for three days on 10 cm Falcon 3003 dishes in DMEM/F12 medium (Gibco BRL) without phenol red supplemented with 10% charcoal-stripped FBS. Cells were then plated onto 6-well dishes and expanded overnight to approximately 30% density. Cells were then rinsed with PBS and maintained for transfection in Optimem (Gibco BRL) without further additions. Transfection of 1 µg reporter plus 800 ng of pSV β-galactosidase construct and 50 ng of CXR expression vector was performed using 3ml of LipofectAMINE (Invitrogen) per well, according to the manufacturer protocol. After 24 h incubation, cells were rinsed with PBS and DMEM/F12 containing 10% delipidated/charcoal-stripped FBS containing either drugs or vehicle control was added. After 16 hours induction, cells were rinsed with PBS and lysed in 600 µl CAT lysis buffer and assayed for CAT enzyme using the CAT-ELIZA kit (Roche Molecular Biochemicals). CAT levels were then normalized against β-galactosidase levels to compensate for variations in transfection efficiency.

### Isolation and characterisation of a drug responsive Enhancer of the mouse 5-aminolevulinic acid gene

### Cloning of the 5' flanking region of mALAS1

As only several hundred base pairs sequence information of mALAS1 were known, the strategy of isolating 5' flanking region was "chromosomal walking" by southern blotting. For this purpose two bacterial artificial chromosome (BAC) clones termed 113 and 266 were used (BAC 113d22 and 266n18, mouse C57 B/6 from Genome Systems Inc., St. Louis, MO, USA). By application of said method four different clones, spanning about 17 kb of flanking region were identified. Of these clones a 2.6 kb fragment was "inducible" in reporter gene assays.

The 2.6 kb HindIII fragment (-14.7kb to - 17.3kb) was cloned as follows: 3µg BAC 266 were digested with HindIII over night. A 0.7% gel was run for 6 hours at 90V and the 2.6 kb band was extracted and ligated to HindIII cut and dephosphorylated, gel-purified pBS bluescript (Stratagene, La Jolla, California, USA) and heat-shock transformed. Subcloning into pGL3LUCpro + MCS was done using EcoRI and KpnI, so that the fragment was in forward direction. In luciferase reporter assay using a LMH (Leghorn male hepatoma) cell system said construct showed drug inducibility.

### Identification of drug responsive enhancer sequence (DRES) in the 2.6 kb HindIII fragment

The inventors of the present invention have found that the sequence in the priority application PCT/IB02/01258 designated as 2.6 kb HindIII fragment has acutally a length of 2.8 kb in the genome. The cloning of the fragment in bacteria resulted in the elimination of 0.2 kb without functional consequences.

The 2.6 kb fragment was completely sequenced. The fragment has a length of 2604bp. For easy orientation, numbering was given starting at 1 for the 5' end of said fragment. The following fragments were amplified using the 2.6 kb fragment as template: 280bp fragment (398 to 677) (Seq. Id. No. 4), 321bp fragment (398 to 718) (Seq. Id. No. 5), 328bp fragment (350 to 677) (Seq. Id. No. 6) and 369bp fragment (350 to 718) (Seq. Id. No. 7. The following primer pairs were used:
280bp fragment: mALAS-16.6FwdKpn (Seq. Id. No. 11) and mALAS-16.35rvXhoI (Seq. Id. No. 12).
328bp fragment: mALAS-16.65fwdKpn (Seq. Id. No. 13) and mALAS-16.35rvXhoI (Seq. Id. No. 12).
321bp fragment: mALAS-16.6fwdKpn (Seq. Id. No. 11) and mALAS-16.3rvXhoI (Seq. Id. No. 14).
369bp fragment mALAS-16.65fwdKpn (Seq. Id. No. 13) and mALAS-16.3rvXhoI (Seq. Id. No. 14).

A 175bp fragment (441 to 615) (Seq. Id. No. 3) was isolated by digesting the 2.6kb fragment with SacI/XbaI. All fragments were subcloned in the pGL3LUCpro + MCS vector and the resulting constructs tested for inducibility in LMH reporter gene assay (Fig. 6). All five fragments showed enhanced induction with as inducer Metyrapone 500µM. The 369bp fragment was termed Drug responsive enhancer sequence DRES.

The 369bp DRES sequence and discovered putative nuclear receptor binding sites are shown in figure 8. Putative nuclear receptor binding sites were discovered by MatInspector using a core similarity of 0.8 and matrix similarity of 0.85. Putative binding sites include: upstream stimulatory factor (USF), activator protein 1/ 4 (AP1/ AP4), nuclear factor 1 (NF-1), CAR/ CXR/ PXR (DR4), ets-1, estrogen receptor (ER), RAR-related orphan receptor alpha1 (RORA1), PPAR (DR1), nuclear factor κ B (NFκB), c-Rel, sterol response element binding protein (SREBP), stimulatory protein 1 (SP1). SacI and XbaI are the restriction sites of the 175bp core fragment. Bold arrows indicate 5' and 3' ends of the clones from expanding the core SacI/ XbaI fragment.

### Drug induction pattern of 369bp DRES in LMH luciferase reporter gene assay

To compare the 369bp DRES to other inducible fragments, a series of different drugs was used as inducers: clothrimazole (Clo, 10µM), dexamethasone (Dex, 50µM), glutethimide (GE, 500µM), metyrapone (Met, 500µM), phenobarbital (PB, 500µM), PCN (10µM), propylisopropylacetamide (PIA, 250µM), rifampicin (Rif, 10µM), RU486 (10µM), TCPOBOP (10µM).

Figure 7 shows induction of the 369bp DRES by different drugs. Data shown is from two independent experiments. As it was already observed for the 2.8 kb HindIII clone , metyrapone is the strongest inducer, together with glutethimide and PIA.

### Mutagenesis of the DR4

All experiments done so far, showed that the region containing the DR4 sites was absolutely required to get any induction at all. To show the direct involvement of the DR4 sites (DR4-1 and DR4-2), they were mutated (in the 369bp context) in the following way:

The DR4 halfsites were mutated individually and both together (see figure 9). Mutated base pairs are underlined and italic.

To have convenient analysis tools for succesful mutagenesis, the NR1 halfsite was mutated into a EcoRI site and the NR2 halfsite into a PstI site.

### Mutagenesis/cloning

In a first mutagenesis, each hexamer halfsite was mutated individually. The first PCR was performed using rvp3 plasmid and DR4mt1rv (Seq. Id. No. 33) and glp2 plasmid and DR4mt1fwd (Seq. Id. No. 34) [DR4-2/hs1], DR4mt2rv (Seq. Id. No. 35) and DR4mt2fwd (Seq. Id. No. 36) [DR4-2/hs2] primer pairs, using the 369bp-LUC clone as template. PCR was run out on 1.5% agarose gel and bands were extracted. The second PCR was performed using the PCR products from the first PCR as template (1µl out of 20µl for each product) and running a PCR with the external primer rvp3 and glp2. The PCR was run out on a 1.2% agarose gel and the bands were extracted. Then, a KpnI/ XhoI digestion was performed on that fragment, and after purification directly ligated to KpnI/ XhoI cut pGL3LUCpro + MCS (no gel separation of bands required because after digestion there was only one fragment with the compatible sticky ends for cloning) and heat-shock transformed. Minipreps of DNA were analysed by KpnI/ XhoI digestion (insert) and XhoI/ EcoRI (DR4-2/hs1, check for mutation) or XhoI/ PstI (DR4-2/hs2, check for mutation) digestion. Finally, clones were sequenced to confirm desired mutation and no other bp mutations.

In a second step, both halfsites were mutated. For the double mutant DR4-2/hs1,2, procedure was like above, only that the 369DR4mt2-LUC was taken as template for the first PCR and that the primers DR4mt2mtlfwd (Seq. Id. No. 37) and DR4mt2mtlrv (Seq. Id. No. 38) were used. This construct is shortly termed 369DR4mut-LUC.

### Inducibility in LMH reporter gene assay

Figure 10 shows induction of DR4 mutant constructs of mouse 369 DRES in luciferase reporter gene assay in LMH cells. As inducer metyrapone 500µM was used. Data shown is from three independent experiments.

### Inducibility of the ALA synthase drug responsive enhancer sequence by mouse CAR and mouse PXR in transactivation assays.

### Figure 14: Transactivations

To test the effects of the mutations on transcriptional activation, transactivations in CV-1 cells with mouse PXR and mouse CAR and their respective inducers PCN and TCPOBOP, were performed (Fig. 14). The inducible DNA constructs were subcloned into the CAT5 reporter vector and tested for their ability to be transactivated by nuclear receptor transcription factors in CV-1 cells. Mouse PXR (A) and mouse CAR (B) coding regions cloned into the pSG5 expression vector were cotransfected along with a vector expressing pSV β-galactosidase as control. Cells were then treated for 16h with either drugs (A, PCN; B, TCPOBOP) or vehicle control and cell extracts were analyzed for CAT expression normalized against β-galactosidase levels. Experiments were repeated at least three times and error bars represent standard deviations.

As seen in Figure 14A, the single mutations in the DR4-1 binding site reduced the induction by PCN to 2.7-fold and 2.0-fold, respectively, while the double mutant exhibited 1.9-fold activation. The single mutations in DR4-2 did not significantly reduce induction relative to the wild type value of 5.6-fold, displaying 5.8- and 5.3-fold activation, respectively, whereas the double mutant was reduced to 2.5-fold induction. The alteration of both NR binding sites in the 369bp element resulted in the elimination of PXR-mediated drug response. In Figure 14B, CAR transactivations of the DR4-1 single and double mutants did not exhibit any response to TCPOBOP, with even the basal CAR activity eliminated. In comparison, the DR4-2/hs1 and DR4-2/hs2 single mutants still exhibited basal expression that exceeded androstanol-treated levels by 2.5- and 1.9-fold, respectively. TCPOBOP induction exceeded basal levels in the DR4-2/hs1 construct at a modest 1.4 fold, whereas no induction was observed for either the DR4-2/hs2 construct or the double mutant. For the DR4-1,2/hs1,2 quadruple mutant, no basal increase in CAT expression was observed in the absence of androstanol and the induction capacity was completely eliminated.

Both DR4-1 and DR4-2 sites in the 369bp ADRES element were found to be required for full activation by either PCN or TCPOBOP. Together, the data indicate that DR4-1 is essential for NR-mediated induction via CAR or PXR, whereas DR4-2 might contribute in a more indirect fashion to the overall activation of the 369bp ADRES. These studies demonstrate an essential contribution of the sequences within the putative DR4 NR binding sites to drug induction of the ADRES elements.

### Gel shift assays to determine where mouse RXR and mouse CAR bind within the enhancer

As both DR4-1 and DR4-2 NR binding sites clearly contribute to the transcriptional activation exhibited by the ADRES elements in transactivations, gel-mobility shift assays were used to determine where PXR and CAR might bind within the enhancer (Fig. 15). Mouse CAR, PXR and RXR proteins were expressed using the TNT T7 Quick Coupled Translation System (Promega) according to the manufacturer's protocol. For DNA fragment labeling, ends were filled in with the Klenow fragment of *E*. *coli* DNA polymerase I in the presence of radiolabeled [α-³²P]ATP and purified over a Biospin 6 chromatography column. A volume of labeled oligonucleotide corresponding to 100,000 cpm was used for each reaction in 10 mM Tris-HCl (8.0) / 40 mM KCl / 0.05% Nonidet P40 / 6% glycerol (vol./vol.) / 1 mM DTT containing 0.2 µg of poly(dI-dC) and 2.5 µl in-vitro synthesized proteins as described previously (10,12). The reaction mix was incubated for 20 min at room temperature and electrophoresed on a 6% polyacrylamide gel in 0.5X Tris / borate / EDTA buffer followed by autoradiography.

The 369bp wild type and mutant constructs were examined in the presence of mouse RXR and either mouse PXR (Fig. 15A) or mouse CAR (Fig. 15B). *In* vitro transcribed/translated mouse RXR or mouse PXR alone bound to the ³²P-radiolabeled 369bp ADRES (Fig. 15A, lanes 1 and 2), whereas PXR/RXR heterodimers bind the wild type drug responsive enhancer (Fig. 15A, lane 3). PXR/RXR binding to the 369bp ADRES element was eliminated in the DR4-1/hs1 and DR4-1/hs2 single mutants as well as the DR4-1/hs1,2 double mutant DNA sequences, as demonstrated by the absence of bands in lanes 4, 5 and 6, respectively. Interestingly, the DR4-2/hs1,2 construct still bound the PXR/RXR heterodimer, as seen in lane 7. Moreover, the binding of PXR/RXR heterodimers was absent with the DR4-1,2/hs1,2 quadruple mutant (Lane 8). In a similar fashion, mouse CAR and RXR were unable to bind as homodimers to the 369bp ADRES, whereas a shift is observed in the presence of RXR/CAR heterodimers (Fig. 15B, lanes 1-3). CAR/RXR binding is also eliminated in the DR4-1/hs1 and DR4-1/hs2 single mutants as well as the DR4-1/hs1,2 double mutant (lanes 4-6, respectively) but still present in the DR4-2/hs1,2 double mutant (lane 7). Not unexpectedly, the DR4-1,2/hs1,2 quadruple mutant does not bind CAR/RXR heterodimers as observed in lane 8. In summary, these findings demonstrate interactions of PXR/RXR and CAR/RXR heterodimers with the DR4-1 but not the DR4-2 binding sites in the 369bp ADRES elements.

### Drug regulation of the human housekeeping ALA-synthase (ALAS1) gene

Using a computer-assisted screening approach and sequence information publicly available, several regions of the 5' flanking region of the ALAS1 were defined as potential mediators of drug-induction of the human housekeeping ALAS gene via nuclear receptors. The defined regions were isolated from a BAC clone containing 30kb of the upstream region of this gene and introduced into a reporter vector containing the firefly luciferase gene as a reporter for gene activation. These constructs were tested in LMH chicken hepatoma cells, the.only known continuously dividing cell line retaining drug-mediated induction.

In these initial screening approaches, two regions were defined which responded to prototypic inducer drugs.

The better-characterized of these regions was called hA795 (Seq. Id. No. 9) and lies approximately 20kb upstream from the transcriptional start site. By dissecting this fragment, we were able to define a short element, 174bp (Seq. Id. No. 8) in size, which is sufficient to confer induction in LMH cells. The element was termed hA174. Within this hA174 fragment, a DR-4 type nuclear receptor recognition site was found to be necessary for drug induction in site-directed mutagenesis experiments.

The second drug-inducible fragment was called hA8 (Seq. Id. No. 10), is 917 bp in length and is located approximately 16kb upstream from the transcriptional start site. It contains at least predicted DR-3 and DR-4-type nuclear receptor response elements.

In transactivation assays in a heterologous cell line (monkey kidney CV-1 cells) and human hepatoma HepG2 cells, we assessed which nuclear receptor conveys the observed activation. The two candidate receptors pregnane X receptor (PXR) and constitutive androstane receptor (CAR) were tested on these fragments. With both fragments and the hA174 subfragment, induction by human PXR and activation by mouse and human CAR was observed. The mCAR induced activity could be repressed to approximately 50% by the inverse agonist 3α-androstenol and this repression was reversible by the direct agonist TCPOBOP (1,4-bis[2-(3,5-dichloropyridyloxy)]benzene).

### Results

### Drug-induction of different fragments of the human ALAS1 gene.

Fragments were cloned into the pGL3 luciferase reporter vector (Promega Corp) and tested for inducibility by the prototypical hALAS1 inducers phenobarbital (PB) and propylisopropylacetamid (PIA). Four hours after transfection of LMH cells with the constructs, cells were exposed to the drugs for 24 hours, after which luciferase activity was assayed. Results were normalized for transfection efficiency by assaying for activity of co-transfected β-galactosidase. Data shown is one representative experiment (Figure 11).

### The effect of drugs on the hA795 element depends on the presence of a DR-4 motif.

Within the sequence of the hA795 fragment (Seq. Id. No. 9), a putative DR-4 type nuclear receptor response element was found by computer analysis. Site-directed mutagenesis of this element abolished inducibility of this fragment in reporter gene assays in LMH cells. Experiments were performed as described above (Figure 12).

### A core sequence spanning the DR-4 element is sufficient to mediate drug induction in LMH cells.

From the hA795 fragment, the hA174 fragment was derived (Seq. Id. No. 8). It is 174bp in length and within its sequence, the DR-4 response element is contained. Direct repeats of the wildtype hA174 or a mutant, where the DR-4 was destroyed were cloned into the pGL3 reporter vector and tested in LMH cells (Fig. 13). Data is from one representative experiment.

Figure 16 shows the hA174 core element derived from the hA795 fragment conferring drug-mediated transcriptional activation to a reporter gene in LMH cells. We investigated in more detail the contribution of various response element half-sites to drug-induction mediated by this element. Within the hA174 element, an arrangement of three nuclear receptor response element half-sites was found, which lead to the prediction of a DR-4 type, as well as a DR-5 type response element (cf. Figure 18A). By site-directed mutagenesis, we examined the involvement of each of these half-sites to drug induction. In the context of the 2xhA174 reporter construct, each single half-site was mutated in both copies of the hA174 element. The wild-type and mutant constructs were subsequently tested for reporter gene activation in LMH cells as described above. Cells were induced with 400µM phenobarbital (PB) or 250µM propylisopropylacetamid (PIA). As a control, vehicle alone (0.1% DMSO) was added to the cells. After 24 hours, luciferase activity was assayed and normalized against co-transfected β-galactosidase activity in order to compensate for transfection efficiency. Mutagenesis of the HS1 or HS2 half-site leads to abolishment of inducibility of this fragment, whereas alteration of the HS3 half-site merely reduces induction levels. From this, we conclude that the DR-4 element, consisting of the HS1 and HS2 half-sites, is essential for drug induction. The change in induction observed by mutagenesis of the HS3 half-site may indicate a modulatory role for the half-site alone or as a DR-5 in combination with HS2 in drug induction. Data shown are the mean +1SD of three independent experiments.

Figure 17 shows the sub-fragmentation of the hA8 drug-responsive element (Seq. Id. No. 10).

In order to define a core sequence retaining inducibility, the hA8 fragment (Seq. Id. No. 10), which carries PCR-introduced *KpnI* and *Xho*I restriction sites at its ends, was digested with *Sac*I and *NsiI.* The resulting four fragments, a 100bp *Kpn*I/*Sac*I fragment, a 374bp *Sac*I/*Nsi*I fragment, a 240bp *Nsi*I/*Nsi*I fragment, and a 231bp *Nsi*I/*Xho*I fragment, were cloned into a reporter gene vector and subsequently tested for drug induction in LMH cells. Of these fragments, only the 240bp fragment (called hA240) (Seq. Id. No. 39) was able to convey transcriptional activation in response to drugs. Within the core hA240 fragment, we then mutated both half-sites of a predicted DR-4 element (cf. Figure 18B) and observed the effect of this mutation on drug induction (240mutDR4). Alteration of the DR4 element half-sites abolished drug induction in LMH cell reporter gene assays. Data shown are the mean +1SD of three independent experiments.

Figure 18 depicts in detail the core elements of the two drug-responsive regions within the human ALAS1 gene. The position numbers refer to the position of these sequences in the human genome, relative to the transcriptional start site of the human ALAS1 transcript. Response elements are boxed in grey, and individual half-sites are marked with arrows above and, if necessary for distinction, labels below the sequence. In A, the hA174bp core (Seq. Id. No. 8) is depicted. The grey box indicates the DR-4/DR-5 site cluster, made up of three individual half-sites. In B, the 240bp core is shown (Seq. Id. No. 39), with a grey box and arrows marking the functional DR-4 element.

Figure 19 depicts the results of an electrophoretic mobility shift assay, assaying the ability of human PXR and human CAR to bind to the hA174 and hA240 elements. Binding of these nuclear receptors happens in a heterodimeric state with the human 9-cis retinoic acid receptor RXRα. From expression plasmids encoding human RXRα, RXR or CAR, receptor protein was made in vitro using the TNT reticulocyte lysate coupled transcription/translation system (Promega Corp.).

Figure 19A shows an assay using radiolabeled hA174 wildtype fragment or hA174 fragment, where half-site 2 was mutated (cf. Figure 18A). Lanes 1-4: Wildtype hA174 was incubated with no receptor (mock transcription/translation) or with RXRα, PXR or CAR alone. No complex is observed using single receptors. Lanes 5 and 7: Wild-type hA174 was incubated with RXRα and PXR or RXRα and CAR, upon which complex formation between radiolabeled probe and protein is observed. Lanes 6 and 8: Same conditions as in lanes 5 and 7, but instead of wild-type hA174, a radiolabeled probe was used where the half-site 2 was mutated. No complex is formed anymore.

Fig. 19B: Instead of the hA174 fragment, wild-type or DR4-mutant hA240 fragment was radiolabeled and used as probe. Lanes 1-4: Wildtype hA240 was incubated with no receptor (mock transcription/translation) or with RXRα, PXR or CAR alone. No complex is observed using single receptors. Lanes 5 and 7: Wild-type hA240 was incubated with RXRα and PXR or RXRα and CAR, upon which complex formation between radiolabeled probe and protein is observed. Lanes 6 and 8: Same conditions as in lanes 5 and 7, but instead of wild-type probe, radiolabeled hA240DR4(2) mutant was used. No complex is formed.

The abbreviations used are: ALAS, 5-aminolevulinic acid synthase; ADRES, aminolevulinic acid drug responsive enhancer sequence; PB, phenobarbital; DR, hexamer half-site direct repeat; h, hours; bp, basepairs; LMH, leghorn male hepatoma; kb, kilobases; NF1, nuclear factor 1; CYP, cytochrome(s) P450; CXR, chicken xenobiotic receptor; PXR, pregnane X receptor; CAR, constitutive androstane receptor; RXR, 9-*cis*-retinoic acid receptor; PIA, propylisopropylacetamide; PCN, 5-pregnen-3β-ol-20-one-16α-carbonitrile; TCPOBOP, 1,4-bis[2-(3,5-dichloropyridyl-oxy)]benzene; LUC, luciferase; mifepristone, RU-486; clotrimazole, 1-[o-chlorotrityl]-imidaszole; EMSA, electrophoretic mobility shift assay; cpm, counts per minute; FCS, fetal calf serum.

While there are shown and described presently preferred embodiments of the invention, it is to be distinctly understood that the invention is not limited thereto but may be otherwise variously embodied and practiced within the scope of the following claims.

### References

1. **Boshart, M., M. Kluppel, A. Schmidt, G. Schutz, and B. Luckow.** 1992. Reporter constructs with low background activity utilizing the cat gene. Gene **110:**129-30.
2. **Handschin, C., M. Podvinec, and U. A. Meyer.** 2000. CXR, a chicken xenobiotic-sensing orphan nuclear receptor, is related to both mammalian pregnane X receptor (PXR) and constitutive androstane receptor (CAR). Proc Natl Acad Sci U S A **97**:10769-74.
**3. Honkakoski, P., I. Zelko, T. Sueyoshi, and M. Negishi.** 1998. The nuclear orphan receptor CAR-retinoid X receptor heterodimer activates the phenobarbital-responsive enhancer module of the CYP2B gene. Mol Cell Biol **18**:5652-8.
4. **Iniguez-Lluhi, J. A., D. Y. Lou, and K. R. Yamamoto.** 1997. Three amino acid substitutions selectively disrupt the activation but not the repression function of the glucocorticoid receptor N terminus. J Biol Chem **272**:4149-56.
5. **Kim, J., G. Min, and B. Kemper.** 2001. Chromatin assembly enhances binding to the CYP2B1 Phenobarbital-responsive unit (PBRU) of nuclear factor 1, which binds simultaneously with constitutive androstane receptor (CAR)/retinoid X receptor (RXR) and enhances CAR/RXR-mediated activation of the PBRU. J Biol Chem **276**:7559-7567.
6. **Kliewer, S. A., J. M. Lehmann, and T. M. Willson.** 1999. Orphan nuclear receptors: shifting endocrinology into reverse. Science **284**:757-60.
7. **Kliewer, S. A., J. T. Moore, L. Wade, J. L. Staudinger, M. A. Watson, S. A. Jones, D. D. McKee, B. B. Oliver, T. M. Willson, R. H. Zetterstrom, T. Perlmann, and J. M. Lehmann.** 1998. An orphan nuclear receptor activated by pregnanes defines a novel steroid signaling pathway. Cell **92**:73-82.
8. **Lehmann, J. M., D. D. McKee, M. A. Watson, T. M. Willson, J. T. Moore, and S. A. Kliewer.** 1998. The human orphan nuclear receptor PXR is activated by compounds that regulate CYP3A4 gene expression and cause drug interactions. J Clin Invest **102**:1016-23.
9. **Rupp, R. A., U. Kruse, G. Multhaup, U. Gobel, K. Beyreuther, and A. E. Sippel.** 1990. Chicken NFI/TGGCA proteins are encoded by at least three independent genes: NFI-A, NFI-B and NFI-C with homologues in mammalian genomes. Nucleic Acids Res **18**:2607-16.
10. **Savas, U., K. J. Griffin, and E. F. Johnson.** 1999. Molecular mechanisms of cytochrome P-450 induction by xenobiotics: An expanded role for nuclear hormone receptors. Mol Pharmacol **56:**851-7.
11. **Waxman, D. J.** 1999. P450 gene induction by structurally diverse xenochemicals: central role of nuclear receptors CAR, PXR, and PPAR. Arch Biochem Biophys **369**:11-23.

## Claims

1. An isolated nucleic acid comprising at least a DR-4 nuclear receptor binding site wherein said nucleic acid functions as a transcriptional enhancer of the 5-aminolevulinic acid synthase 1 gene and wherein said nucleic acid comprises a sequence selected from the group consisting of SEQ. ID. NOS: 1-7.

2. The nucleic acid of claim 1, wherein said nucleic acid comprises the sequence set forth in SEQ. ID. NO: 1.

3. The nucleic acid of claim 1 or 2, wherein said nucleic acid mediates chemical compound induced transcriptional activation.

4. The nucleic acid of claim 3, wherein said chemical compound is a candidate compound for therapeutical use or a drug.

5. The nucleic acid of claims 1, 3 or 4, wherein said nucleic acid comprises a sequence selected from the group consisting of SEQ. ID. NOS. 2-7.

6. A genetic construct comprising a nucleic acid having a sequence selected from the group consisting of SEQ. ID. NOS. 1- 8, 10 and 39, operably linked to a nucleic acid encoding a reporter molecule.

7. The genetic construct of claim 6 , wherein said reporter molecule has an enzymatic activity.

8. The genetic construct of claim 7, wherein said reporter molecule activity can be detected by colorimetry, radioactivity, fluorescence or chemiluminescence.

9. The genetic construct of any one of claims 6-8, wherein said reporter molecule is selected from the group consisting of luciferase, beta-galactosidase, chloroamphenicol acetyltransferase, alkaline phosphatase and green fluorescent protein.

10. A method for testing compounds for modulation of heme and/or P450 cytochrome synthesis comprising contacting suitable cells comprising a genetic construct according to claims 6-9 with a test compound and detecting enhanced or repressed expression and/or transcription of the nucleic acids sequence encoding the reporter gene.

11. The method of claim 10, wherein said compound is a candidate drug for therapeutical use or a drug.

12. The method of claims 10 or 11, wherein enhanced expression of the nucleic acid sequence encoding the reporter gene is detected by colorimetry, fluorescence, radioactivity or chemiluminescence.

13. The method of any one of claims 10-12, wherein enhanced transcription of the nucleic acid encoding the reporter gene is detected by quantitative PCR.

14. The method of any one of claims 10 -13, wherein said cells are Leghorn Male Hepatoma (LMH) cells, other hepatoma cells, monkey kidney cells (CV-1, COS-1) or human kidney cells.

15. Use of a nucleic acid sequence of any one of claims 1 - 5 for the testing of chemical compounds as modulators of heme and/or P450 cytochrome synthesis.

16. Use of a nucleic acid having a sequence selected from the group consisting of Seq. Id. No. 8 to 10 and 39 for the testing of chemical compounds as modulators of heme and/or P450 cytochrome synthesis.

17. Use of a genetic construct of any one of claims 6-9 for the testing of chemical compounds as modulators of heme and/or P450 cytochrome synthesis.

## Patentansprüche

1. Isolierte Nukleinsäure, umfassend wenigstens eine DR-4-Bindungsstelle für nukleäre Rezeptoren, wobei die Nukleinsäure die Funktion eines transkriptionalen Enhancers des Gens der 5-Aminolävulinsäure-Synthase 1 besitzt und wobei die Nukleinsäure eine aus der Gruppe, bestehend aus SEQ. ID. NOS: 1-7, ausgewählte Sequenz umfaßt.

2. Nukleinsäure nach Anspruch 1, die die in SEQ. ID. NO:1 angegebene Sequenz umfaßt.

3. Nukleinsäure nach Anspruch 1 oder 2, die eine durch eine chemische Verbindung induzierte transkriptionale Aktivierung vermittelt.

4. Nukleinsäure nach Anspruch 3, wobei es sich bei der chemischen Verbindung um eine Kandidatenverbindung zur therapeutischen Verwendung oder einen Arzneistoff handelt.

5. Nukleinsäure nach Anspruch 1, 3 oder 4, die eine aus der Gruppe, bestehend aus SEQ. ID. NOS: 2-7, ausgewählte Sequenz umfaßt.

6. Gentechnisches Konstrukt, umfassend eine Nukleinsäure mit einer aus der Gruppe, bestehend aus SEQ. ID. NOS: 1-8, 10 und 39, ausgewählten Sequenz in operativer Verknüpfung mit einer für ein Reportermolekül codierenden Nukleinsäure.

7. Gentechnisches Konstrukt nach Anspruch 6, wobei das Reportermolekül eine enzymatische Aktivität aufweist.

8. Gentechnisches Konstrukt nach Anspruch 7, wobei sich die Reportermolekülaktivität mittels Kolorimetrie, Radioaktivität, Fluoreszenz oder Chemilumineszenz nachweisen läßt.

9. Gentechnisches Konstrukt nach einem der Ansprüche 6-8, wobei das Reportermolekül aus der Gruppe Luciferase, beta-Galactosidase, Chloramphenicol-Acetyltransferase, alkalische Phosphatase und grünes Fluoreszenzprotein ausgewählt ist.

10. Verfahren zum Testen von Verbindungen auf eine Modulation der Häm- und/oder P450-Cytochrom-Synthese, bei dem man geeignete, ein gentechnisches Konstrukt nach Ansprüchen 6-9 umfassende Zellen mit einer Testverbindung in Kontakt bringt und eine verstärkte oder reprimierte Expression und/oder Transkription der für das Reportergen codierenden Nukleinsäuresequenz nachweist.

11. Verfahren nach Anspruch 10, wobei es sich bei der Verbindung um eine Kandidatenverbindung zur therapeutischen Verwendung oder einen Arzneistoff handelt.

12. Verfahren nach Anspruch 10 oder 11, wobei eine verstärkte Expression der für das Reportergen codierenden Nukleinsäuresequenz mittels Kolorimetrie, Fluoreszenz, Radioaktivität oder Chemilumineszenz nachgewiesen wird.

13. Verfahren nach einem der Ansprüche 10-12, wobei eine verstärkte Transkription der für das Reportergen codierenden Nukleinsäure mittels quantitativer PCR nachgewiesen wird.

14. Verfahren nach einem der Ansprüche 10-13, wobei es sich bei den Zellen um LMH (Leghorn Male Hepatoma)-Zellen, andere Hepatomzellen, Affennierenzellen (CV-1, COS-1) oder menschliche Nierenzellen handelt.

15. Verwendung einer Nukleinsäuresequenz nach einem der Ansprüche 1-5 zum Testen chemischer Verbindungen als Modulatoren der Häm- und/oder P450-Cytochrom-Synthese.

16. Verwendung einer Nukleinsäuresequenz mit einer aus der Gruppe, bestehend aus Seq. Id. Nr. 8 bis 10 und 39, ausgewählten Sequenz zum Testen chemischer Verbindungen als Modulatoren der Häm- und/oder P450-Cytochrom-Synthese.

17. Verwendung eines gentechnischen Konstrukts nach einem der Ansprüche 6-9 zum Testen chemischer Verbindungen als Modulatoren der Häm- und/oder P450-Cytochrom-Synthese.

## Revendications

1. Acide nucléique isolé comprenant au moins un site de liaison à un récepteur nucléaire DR-4, dans lequel ledit acide nucléique fonctionne comme amplificateur transcriptionnel du gène de l'acide 5-aminolévulinique synthétase 1 et dans lequel ledit acide nucléique comprend une séquence choisie dans le groupe composé des SEQ ID n° : 1 à 7.

2. Acide nucléique selon la revendication 1, dans laquelle ledit acide nucléique comprend la séquence indiquée à la SEQ ID n° : 1.

3. Acide nucléique selon la revendication 1 ou 2, dans laquelle ledit acide nucléique effectue la médiation d'une activation transcriptionnelle induite par un composé chimique.

4. Acide nucléique selon la revendication 3, dans laquelle ledit composé chimique est un composé candidat pour une utilisation thérapeutique ou une substance médicamenteuse.

5. Acide nucléique selon la revendication 1, 3 ou 4, dans laquelle ledit acide nucléique comprend une séquence choisie dans le groupe composé des SEQ ID n° : 2 à 7.

6. Construit génétique comprenant un acide nucléique ayant une séquence choisie dans le groupe composé des SEQ ID n° : 1 à 8, 10 et 39, liée opérationnellement à un acide nucléique codant pour une molécule rapporteuse.

7. Construit génétique selon la revendication 6, dans laquelle ladite molécule rapporteuse a une activité enzymatique.

8. Construit génétique selon la revendication 7, dans laquelle ladite activité de la molécule rapporteuse peut être détectée par : colorimétrie ; radioactivité ; fluorescence ; ou chimiluminescence.

9. Construit génétique selon l'une quelconque des revendications 6 à 8, dans laquelle ladite molécule rapporteuse est choisie dans le groupe composé de : la luciférase ; la bêta-galactosidase ; la chloramphénicol acétyltransférase ; la phosphatase alcaline ; et la protéine fluorescente verte.

10. Procédé pour tester des composés destinés à une modulation de la synthèse de l'hème et/ou du cytochrome P450 comprenant les étapes consistant à : mettre en contact des cellules appropriées, comprenant un construit génétique, selon les revendications 6 à 9, avec un composé de test ; et détecter une expression et/ou une transcription amplifiée ou réprimée de la séquence d'acide nucléique codant pour le gène rapporteur.

11. Procédé selon la revendication 10, dans lequel ledit composé est une substance médicamenteuse candidate pour une utilisation thérapeutique ou une substance médicamenteuse.

12. Procédé selon la revendication 10 ou 11, dans lequel une expression amplifiée de la séquence d'acide nucléique codant pour le gène rapporteur est détectée par : colorimétrie ; fluorescence ; radioactivité ; ou chimiluminescence.

13. Procédé selon l'une quelconque des revendications 10 à 12, dans lequel une transcription amplifiée de l'acide nucléique codant pour le gène rapporteur est détectée par une ACP quantitative.

14. Procédé selon l'une quelconque des revendications 10 à 13, dans lequel lesdites cellules sont des cellules d'hépatome de mâle Leghorn (LMH), d'autres cellules d'hépatome, des cellules de rein de singe (CV-1, COS-1) ou des cellules de rein humain.

15. Utilisation d'une séquence d'acide nucléique, selon l'une quelconque des revendications 1 à 5, pour le test de composés chimiques comme modulateurs de la synthèse de l'hème et/ou du cytochrome P450.

16. Utilisation d'un acide nucléique ayant une séquence choisie dans le groupe composé des Seq. Id. n° : 8 à 10 et 39 pour le test de composés chimiques comme modulateurs de la synthèse de l'hème et/ou du cytochrome P450.

17. Utilisation d'un construit génétique, selon l'une quelconque des revendications 6 à 9, pour le test de composés chimiques comme modulateurs de la synthèse de l'hème et/ou du cytochrome P450.
